# EUROPEAN PATENT SPECIFICATION

(11) **EP 2 135 078 B1**
(45) Date of publication and mention of the grant of the patent: **21.08.2013**
(21) Application number: 08742040.2
(22) Date of filing: 10.03.2008
(51) Int. Cl.: G01N 33/68, G01N 33/50

(54) **METHODS FOR IDENTIFYING AGENTS AND THEIR USE FOR THE PREVENTION OR STABILIZATION OF FIBROSIS**
VERFAHREN ZUR IDENTIFIZIERUNG VON WIRKSTOFFEN UND IHRE VERWENDUNG ZUR PRÄVENTION ODER STABILISIERUNG VON FIBROSE
MÉTHODES D'IDENTIFICATION D'AGENTS ET LEUR UTILISATION DANS LA PRÉVENTION OU LA STABILISATION DE LA FIBROSE

(30) Priority: 09.03.2007 US 905943 P
(43) Date of publication of application: 23.12.2009
(73) Proprietor: DiscoveRx Corporation, Fremont, CA 94538 (US)
(72) Inventor: KUNKEL, Eric, J., San Mateo, CA 94401 (US); ROSLER, Elen, S., Burlingame, CA 94010 (US); PRIVAT, Sylvie, So. San Francisco, California 94080 (US); MELROSE, Jennifer, E., La Honda California 94080 (US)
(74) Representative: Brasnett, Adrian Hugh
(86) International application number: PCT/US2008/003152
(87) International publication number: WO 2008/112195

(56) References cited:
- WO-A1-2005/093561
- WO-A2-2006/105147
- US-A1- 2003 017 445
- US-A1- 2003 113 807
- US-A1- 2003 138 811
- US-B1- 6 801 859
- BIOSEEK: "BioMAP® Systems, Human Cell Systems Biology for Drug Discovery, Facts Sheet" BIOSEEK, 1 July 2005 (2005-07-01), pages 1-10, XP002587186
- ISHII Y ET AL: "Gefitinib prevents bleomycin-induced lung fibrosis in mice" AMERICAN JOURNAL OF RESPIRATORY AND CRITICAL CARE MEDICINE 20060901 US LNKD- DOI:10.1164/RCCM.200509-1534OC, vol. 174, no. 5, 1 September 2006 (2006-09-01), pages 550-556, XP002587187
- WANG S-W ET AL: "Amphiregulin expression in human mast cells and its effect on the primary human lung fibroblasts" JOURNAL OF ALLERGY AND CLINICAL IMMUNOLOGY, MOSBY, INC, US LNKD- DOI:10.1016/J.JACI.2004.11.037, vol. 115, no. 2, 1 February 2005 (2005-02-01), pages 287-294, XP004775675 ISSN: 0091-6749
- SCARPA R C ET AL: "Insulin-like growth factor (IGF) induced proliferation of human lung fibroblasts is enhanced by neurotensin" PEPTIDES, ELSEVIER, AMSTERDAM LNKD- DOI:10.1016/J.PEPTIDES.2005.03.044, vol. 26, no. 11, 1 November 2005 (2005-11-01), pages 2201-2210, XP025378613 ISSN: 0196-9781 [retrieved on 2005-11-01]
- SERLIN D M ET AL: "Interleukin-1[beta] induces osteopontin expression in pulmonary fibroblasts" JOURNAL OF CELLULAR BIOCHEMISTRY 20060215 US LNKD- DOI:10.1002/JCB.20661, vol. 97, no. 3, 15 February 2006 (2006-02-15), pages 519-529, XP002587188
- HUANG M ET AL: "IL-7 inhibits fibroblast TGF-[beta] production and signaling in pulmonary fibrosis" JOURNAL OF CLINICAL INVESTIGATION 2002 US LNKD- DOI:10.1172/JCI200214685, vol. 109, no. 7, 2002, pages 931-937, XP002587189 ISSN: 0021-9738

## Description

### FIELD OF THE INVENTION

The present invention provides methods for identifying agents that stabilize or reverse fibrosis and relates to the fields of biology, molecular biology, chemistry, medicinal chemistry, pharmacology, and medicine.

### BACKGROUND

Knowledge of the biochemical pathways by which cells detect and respond to stimuli is important for the discovery, development, and correct application of pharmaceutical products. Cellular physiology involves multiple pathways, which have complex relationships. For example, pathways split and join; there are redundancies in performing specific actions; and response to a change in one pathway can modify the activity of another pathway, both within and between cells. In order to understand how a candidate agent is acting and whether it will have the desired effect, the end result, and effect on pathways of interest is as important as knowing the target protein.

Fibrosis is the formation or development of excess fibrous connective tissue in an organ or tissue as a reparative or reactive process, as opposed to formation of fibrous tissue as a normal constituent of an organ or tissue. Inflammation resolution and fibrosis are inter-related conditions with many overlapping mechanisms, where macrophages, T helper cells, and myofibroblasts each play important roles in regulating both processes. Following tissue injury, an inflammatory stimulus is often necessary to initiate tissue repair, where cytokines released from resident and infiltrating leukocytes stimulate proliferation and activation of myofibroblasts. However, in many cases this drive stimulates an inappropriate pro-fibrotic response. In addition, activated myofibroblasts can take on the role of traditional APCs, secrete pro-inflammatory cytokines, and recruit inflammatory cells to fibrotic foci, amplifying the fibrotic response in a vicious cycle.

Among the many pathologic conditions associated with fibrosis are included, without limitation, pulmonary fibrosis, renal fibrosis, hepatic fibrosis, cardiac fibrosis, and systemic sclerosis. Fibrotic processes in epithelial tissues (i.e. lung, liver, kidney and skin) share many of the same mechanisms and features, particularly epithelial-fibroblast cross-talk, while fibrosis of non-epithelial tissues (i.e. the heart, nervous tissue, bone marrow) appears to be somewhat different.

Renal fibrosis is the inevitable consequence of an excessive accumulation of extracellular matrix that occurs in virtually every type of chronic kidney disease. The pathogenesis of renal fibrosis is a progressive process that ultimately leads to end-stage renal failure, a devastating disorder that requires dialysis or kidney transplantation. In a simplistic view, renal fibrosis represents a failed wound-healing process of the kidney tissue after chronic, sustained injury. Several cellular pathways, including mesangial and fibroblast activation as well as tubular epithelial-mesenchymal transition, have been identified as the major avenues for the generation of the matrix-producing cells in diseased conditions.

Pulmonary fibrosis is characterized by lung inflammation and abnormal tissue repair, resulting in the replacement of normal functional tissue with an abnormal accumulation of fibroblasts and deposition of collagen in the lung. This process involves cellular interactions via a complex cytokine-signaling mechanism and heightened collagen gene expression, ultimately resulting in its abnormal collagen deposition in the lung. In addition to inflammatory cells, the fibroblast and signaling events that mediate fibroblast proliferation and myofibroblasts play important roles in the fibrotic process. However, the most potent anti-inflammatory drugs that have been widely used in the treatment of pulmonary fibrosis do not seem to interfere with the fibrotic disease progression.

Hepatic fibrosis is an accumulation in the liver of connective tissue in response to hepatocellular damage of nearly any cause. It results from excessive production or deficient degradation of the extracellular matrix. Fibrosis itself causes no symptoms but can lead to portal hypertension or cirrhosis.

Systemic sclerosis is a chronic disease of unknown cause characterized by diffuse fibrosis, degenerative changes, and vascular abnormalities in the skin, joints, and internal organs (especially the esophagus, lower GI tract, lung, heart, and kidney). Common symptoms include Raynaud's phenomenon, polyarthralgia, dysphagia, heartburn, and swelling and eventually skin tightening and contractures of the fingers. Lung, heart, and kidney involvement accounts for most deaths. Specific treatment is difficult, and emphasis is often on treatment of complications.

A variety of drugs have been tried in various fibroses, particularly lung fibrosis, with very little success. Anti-inflammatory drugs including prednisolone and azathioprine have little effect on fibrosis suggesting that inflammation is only the initiator, but not the driver of the disease. The use of non-specific anti-proliferatives like colchicine and cyclophosphamide will also prevent repair of the fibrotic tissue by impairing e.g. epithelial growth. Treatment with IFN-γ has shown some utility but is limited by severe side effects. Therefore, there is a need to identify potential therapeutics with the ability of selectively stop fibrotic processes while allowing the physiological healing of the tissue.

WO 2006/105147 describes the biological dataset profiling of cardiovascular disease and cardiovascular inflammation, where datasets comprising information for multiple cellular parameters are compared and identified, and used in the evaluation of candidate pharmacologic agents for suitability as therapeutic agents.

WO 2005/093561 describes methods and systems for evaluating biological dataset profiles relating to asthma and other atopic conditions, where datasets comprising information for multiple cellular parameters are compared and identified, and used in the evaluation of candidate pharmacologic agents for suitability as therapeutic agents.

Ishii et al. (American Journal of Respiratory and Critical Care Medicine, vol. 174, no. 5, 2006, pages 550-556) presents findings suggesting that, in the preclinical setting, tyrosine kinase inhibitors of the epidermal growth factor receptor may have a protective effect on lung fibrosis induced by bleomycin.

Wang et al. (Journal of Allergy and Clinical Immunology, vol. 115, no. 2, 2005, pages 287-294) describes the secretion of amphiregulin by human cord blood-derived mast cells stimulated with IgE cross-linking and the amphiregulin induced proliferation of primary human lung fibroblasts.

Scarpa et al. (Peptides, vol. 26, no. 11, 2005, pages 2201-2210) reports that neurotensin induces proliferation of human lung fibroblasts.

Serlin et al. (Journal of Cellular Biochemistry, vol. 97, no. 3, 2006, pages 519-529) reports that interleukin-1β induces osteopontin expression in pulmonary fibroblasts.

Huang et al. (Journal of Clinical Investigation, vol. 109, no. 7, 2002, pages 931-937) reports that interleukin-7 inhibits fibroblast TGF-β production and signalling in pulmonary fibrosis.

Human primary cell-based assay systems (BioMAP^{®} Systems) that model in vitro the complex biology of human disease, including biology relevant to inflammation and fibrosis, and which can be used for screening and development of drugs eliciting complex biological activities, have been developed: see U.S. Patent Nos. 6,656,695 and 6,763,307 and PCT publication Nos. 01/67103, 03/23753, 04/22711, 04/63088, 04/94609, 05/23987, 04/94992, 05/93561. BioMAP Systems are capable of detecting and distinguishing activities of a broad range of mechanistically diverse compound classes, including anti-proliferative drugs, immunosuppressive drugs, anti-inflammatory drugs etc. For example, see Kunkel et al. (2004) Assay Drug Dev Technol. 2:431-41; and Kunkel et al. (2004). FASEB J. 18:1279-81. A fact sheet describing the use of BioMAP® systems for drug discovery was published by the applicant in July 2005.

Activity profiling of compounds, including experimental compounds as well as drugs approved for human or veterinary use in BioMAP Systems provides an enhanced understanding of the mechanism of action of compounds and allows the identification of compounds that are suitable for a particular therapeutic use, based on the favorable combinations of biological activities which these compounds induce in BioMAP Systems.

Methods and systems for the discovery and evaluation of compound as treatment for fibrosis are of great interest. The present invention addresses this issue.

### SUMMARY OF THE INVENTION

The present invention provides a method for identifying agents that stabilize or reverse fibrosis according to claim 1.

A typical biological dataset profile comprises readouts from changes in multiple cellular parameters resulting from exposure of cells to biological factors in the absence or presence of a candidate agent, where the agent may be a chemical agent, e.g. drug candidate; or genetic agent, e.g. expressed coding sequence. Datasets may include control dataset profiles, and/or dataset profiles that reflect the parameter changes of known agents. Known agents may include those having acceptable therapeutic activities against fibrotic disease states as well as those exemplifying undesirable side effects. For analysis of multiple context-defined systems, the output data from multiple systems may be concatenated.

In the method of the invention a candidate agent is tested in a panel of assays, wherein the assays provide readouts from changes in multiple cellular parameters resulting from exposure of multiple different cell types to biological factors" to identify whether the candidate agent possesses a combination of features characteristic of an ideal anti-fibrotic agent, which features include: (1) inhibition of matrix remodeling with promotion of wound healing, (2) protection of epithelial health and growth, (3) controlling provisional fibrin matrix deposition, and (4) selected anti-inflammatory activities. The possession of these features is manifested by specific dataset profiles in the assay results, as described herein.

Agents and combinations of agents identified by the methods of the invention may be formulated with pharmaceutically acceptable excipients for use in the treatment of fibrosis.

### BRIEF DESCRIPTION OF THE DRAWINGS

Figure 1 illustrates cells and factors involved in pulmonary fibrosis.

Figures 2A-C illustrate fibrosis context screening of drugs currently used for treatment of fibrosis.

Figures 3A-B illustrate fibrosis context screening of candidate agents for treatment of fibrosis.

Figure 4 provides an example of desired parameter changes for fibrosis context screening.

### DEFINITIONS

The practice of the present invention will employ, unless otherwise indicated, conventional techniques of molecular biology (including recombinant techniques), microbiology, cell biology, biochemistry and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Handbook of Experimental Immunology" (D. M. Weir & C. C. Blackwell, eds.); "Gene Transfer Vectors for Mammalian Cells" (J. M. Miller & M. P. Calos, eds., 1987); "Current Protocols in Molecular Biology" (F. M. Ausubel et al., eds., 1987); "PCR: The Polymerase Chain Reaction", (Mullis et al., eds., 1994); and "Current Protocols in Immunology" (J. E. Coligan et al., eds., 1991).

The compounds disclosed herein, or their pharmaceutically acceptable salts may contain one or more asymmetric centers and may thus give rise to enantiomers, diastereomers, and other stereoisomeric forms that may be defined, in terms of absolute stereochemistry, as (R)- or (S)- or, as (D)- or (L)- for amino acids. Disclosed herein are all such possible isomers, as well as, their racemic and optically pure forms. Optically active (+) and (-), (R)- and (S)-, or (D)- and (L)- isomers may be prepared using chiral synthons or chiral reagents, or resolved using conventional techniques as known in the art, e.g. by chromatography. When the compounds described herein contain olefinic double bonds or other centers of geometric asymmetry, and unless specified otherwise, it is intended that the compounds include both E and Z geometric isomers. Likewise, all tautomeric forms are also intended to be included.

As used throughout, "modulation" is meant to refer to an increase or a decrease in the indicated phenomenon (e.g., modulation of a biological activity refers to an increase in a biological activity or a decrease in a biological activity).

As used herein, the terms "treatment," "treating," and the like, refer to obtaining a desired pharmacologic and/or physiologic effect. The effect may be prophylactic in terms of completely or partially preventing a disease or condition, or symptom thereof, and/or may be therapeutic in terms of a partial or complete cure for a condition and/or adverse affect attributable to the condition. "Treatment," as used herein, covers any treatment of a disease or condition in a mammal, particularly in a human, and includes: (a) preventing the condition from occurring in a subject which may be predisposed to the condition but has not yet been diagnosed as having it; (b) inhibiting the development of the condition; and (c) relieving the condition, i.e., causing its regression.

An "effective amount" is an amount sufficient to effect beneficial or desired results. An effective amount can be administered in one or more administrations. An effective amount corresponds with the quantity required to provide a desired average local concentration of a particular biologic agent, in accordance with its known efficacy, within the vascular lumen, vascular wall, or other site, for the intended period of therapy. A dose may be determined by those skilled in the art by conducting preliminary animal studies and generating a dose response curve, as is known in the art. Maximum concentration in the dose response curve would be determined by the solubility of the compound in the solution and by toxicity to the animal model, as known in the art.

The terms "individual," "subject," "host," and "patient," used interchangeably herein, refer to a mammal, including, but not limited to, humans, murines, simians, felines, canines, equines, bovines, mammalian farm animals, mammalian sport animals, and mammalian pets. Human subjects are of particular interest.

As used herein, the terms "determining", "assessing", "assaying", "measuring" and "detecting" refer to both quantitative and qualitative determinations and as such, the term "determining" is used interchangeably herein with "assaying," "measuring," and the like. Where a quantitative determination is intended, the phrase "determining an amount" and the like is used. Where either a qualitative or quantitative determination is intended, the phrase "determining a level of proliferation" or "detecting proliferation" is used.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. Unless mentioned otherwise, the techniques employed herein are standard methodologies well known to one of ordinary skill in the art.

It must be noted that as used herein and in the appended claims, the singular forms "a," "and," and "the" include plural referents unless the context clearly dictates otherwise. Thus, for example, reference to "a biomarker" includes a plurality of such biomarkers and reference to "the sample" includes reference to one or more samples and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Moreover any positively recited element of the disclosure provides basis for a negative limitation to exclude that element from the claims.

Further, the dates of publication provided might be different from the actual publication dates that may need to be independently confirmed.

Other objects, features and advantages of the present invention will become apparent from the following detailed description. It should be understood, however, that the detailed description and specific examples, while indicating preferred embodiments of the invention, are given by way of illustration only, since various changes and modifications within the scope of the invention will become apparent to those skilled in the art from this detailed description.

### DETAILED DESCRIPTION OF EMBODIMENTS

The present invention provides a method for identifying agents that stabilize or reverse fibrosis according to claim 1.

By the time a typical patient presents with fibrosis-related symptoms (e.g. difficulty breathing for lung fibrosis, cirrhosis for liver fibrosis, etc.), the fibrosis in the target organ is often quite severe, with much of the target organ architecture having been replaced with extracellular matrix. Stopping this ongoing fibrosis can extend lifespan and improve quality of life. Areas of the target organ where the fibrosis is not extensive may be restored to normal architecture with suitable treatment. To provide these benefits it is desirable for a candidate agent to possess one or more of the features: (1) inhibition of matrix remodeling with promotion of wound healing, (2) protection of epithelial health and growth, (3) controlling provisional fibrin matrix deposition, and (4) selected anti-inflammatory activities.

Conventional treatments for fibrosis consist of anti-inflammatory drugs or strong non-selective anti-proliferatives. The anti-proliferative drugs can inhibit fibroblast growth, but they also prevent re-epithelialization and normalization of the remaining epithelial tissue. At the same time, a large number of non-proliferating fibroblasts producing extracellular matrix are not be affected by these drugs. Thus, there is a need for better anti-fibrosis drugs.

Identification of anti-fibrosis drugs by the methods of the invention utilize datasets of information obtained from biologically multiplexed activity profiling (BioMAP^{®}) of candidate agents. A general description of such methods is provided, for example, in U.S. Patent no. 6,656,695 and U.S. Patent no. 6,763,307; in co-pending U.S. patent applications 10/220,999; 10/236,558; 10/716,349; and 10/856,564. Methods of analysis for such profiles are described in International application PCT/US2004/012688.

Briefly, the methods disclosed herein provide screening assays for biologically active agents, where the effect of altering the environment of cells in culture is assessed by monitoring multiple output parameters. The result is a dataset that can be analyzed for the effect of an agent on a signaling pathway, for determining the pathways in which an agent acts, for grouping agents that act in a common pathway, for identifying interactions between pathways, and for ordering components of pathways.

The methods disclosed herein include characterizing a candidate agent to determine the presence features desirable in an anti-fibrosis agent, including one or more of (1) inhibition of matrix remodeling with promotion of wound healing, (2) protection of epithelial health and growth, (3) controlling provisional fibrin matrix deposition, and (4) selected anti-inflammatory activities, by contacting the candidate agent with cells in a test cell culture with at least two factors acting on said cells in an amount and incubating for a time sufficient to induce a plurality of pathways active in said cell culture; recording changes in at least two parameter readouts associated with said plurality of pathways as a result of introduction of the candidate agent; deriving a dataset of said changes in parameter readouts, comprising data normalized to be a ratio of test to control data on the sample under control conditions in the absence of the candidate agent; and analyzing the dataset by a pattern recognition algorithm to quantify relatedness of said biomap to reference biomaps that include one or more of biomaps of normal cells, cells from similarly diseased tissue, or from cell lines with responses induced by assay combinations involving known pathway stimuli or inhibitors, wherein the presence or absence of relatedness to said reference biomaps provides a characterization of the signaling pathway responsiveness of said patient sample to said therapeutic agent.

In order to identify agents that modulate fibrotic processes, particularly those of relevance to disease, model systems containing cells relevant to fibrosis are used in the screening assays described above. Cells relevant to fibrosis include fibroblasts, e.g. dermal fibroblasts, lung fibroblasts, hepatic fibroblasts, etc., epithelial cells, e.g. bronchial epithelial cells; macrophages, myofibroblasts *etc..* The multi-cell and/or multifactor design of the systems and their analysis through multi-parameter activity profiles work together to optimize information content, enabling rapid but effective analysis of drug and gene target activities in complex cellular responses relevant to clinical disease.

Systems may utilize combinations of cells that are informative of the disease processes, e.g. a combination of fibroblast and mononuclear peripheral blood cells or macrophages; fibroblasts and epithelial cells; *etc*. Cells may be primary cultures or cell lines; and may be from normal tissues or from diseased tissues, e.g. peripheral blood monocytes or fibroblasts from systemic sclerosis patients or pulmonary fibrosis patients may be of interest; and the like. Combinations of exogenous factors may be used to simulate disease conditions.

The present invention provides information regarding an agent's utility in the stabilization and/or reversal of fibrosis. Additional assays may include assays that reveal interactions of other epithelial cells like hepatocytes or renal epithelium with fibroblasts, differential effects of various growth factors stimuli on proliferation of fibroblasts under inflammatory conditions, and additional readouts (e.g other types of extracellular matrix).

The methods of the present invention relate to the identification of novel anti-fibrosis drugs. Agents suitable for testing in the method include, without limitation small molecular compounds, natural products, proteins, peptides, plant or other extracts, in general any agent or substance with biological activity. In one embodiment, the invention is practiced to identify combinations of two or more agents that stabilize and/or reverse fibrosis.

A variety of different candidate agents may be screened by the above methods. Candidate agents encompass numerous chemical classes, though typically they are organic molecules, preferably small organic compounds having a molecular weight of more than 50 and less than about 2,500 daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, nucleic acids, fatty acids, steroids, purines, pyrimidines, derivatives, structural analogs or combinations thereof.

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

Included are pharmacologically active drugs, genetic agents, *etc*. Compounds of interest include chemotherapeutic agents, anti-inflammatory agents, hormones or hormone antagonists, ion channel modifiers, and neuroactive agents. Exemplary of pharmaceutical agents suitable for this invention are those described in, "The Pharmacological Basis of Therapeutics," Goodman and Gilman, McGraw-Hill, New York, New York, (1996), Ninth edition, under the sections: Drugs Acting at Synaptic and Neuroeffector Junctional Sites; Drugs Acting on the Central Nervous System; Autacoids: Drug Therapy of Inflammation; Water, Salts and Ions; Drugs Affecting Renal Function and Electrolyte Metabolism; Cardiovascular Drugs; Drugs Affecting Gastrointestinal Function; Drugs Affecting Uterine Motility; Chemotherapy of Parasitic Infections; Chemotherapy of Microbial Diseases; Chemotherapy of Neoplastic Diseases; Drugs Used for Immunosuppression; Drugs Acting on Blood-Forming organs; Hormones and Hormone Antagonists; Vitamins, Dermatology; and Toxicology. Also included are toxins, and biological and chemical warfare agents, for example see Somani, S.M. (Ed.), "Chemical Warfare Agents," Academic Press, New York, 1992).

Compounds of interest for screening may include known anti-inflammatory drugs, analogs and derivatives thereof, or other modulators of inflammation. Such compounds may include, without limitation: histamine agonists, e.g. histamine, betazole, impromidine; histamine antagonists including H1 selective, H2 selective and non-selective blockers, e.g. doxylamine clemastine, brompheniramine triprolidine, cimetidine, chlorpheniramine, famotidine, diphenhydramine, nizatidine, promethazine, ranitidine, loratidine, levocobastine, cetirizine, acravastine; inhibitors of histamine release, e.g. cromalyn, nedocromil, eicosanoids. Leukotriene antagonists may include zafirlakast; inhibitors of leukotriene synthesis may include zileuton, montelekast, carboprost, dinoprotone, alprostadil, dinoprost, and misoprostol. Kinin modulators include bradykinin and aprotinin. NSAIDs, acetaminophen, aspirin and related salicylates are all of interest. Such drugs may include, without limitation, aspirin and salicylates, meclofenamate, celecoxib, diclofenac sodium, naproxen, rofecoxib, fenoprofen, phenylbutazone, meloxicam, ibuprofen, piroxicam, namebutone, indomethacin, sulindac, ketoprofen, and tometin. Immunosuppressants and anti-proliferatives include rapamycin, methotrexate, azathioprine, cyclosporin, FK-506, cdk inhibitors, and corticosteroids. Statins refer to a known class of HMG-CoA reductase inhibitors. These agents include mevastatin and related compounds, lovastatin (mevinolin) and related compounds, pravastatin and related compounds, simvastatin and related compounds; fluvastatin and related compounds; atorvastatin and related compounds; cerivastatin and related compounds and rosuvastatin.

The method of the invention are ideal for identifying synergistic activities, and the BioMAP systems exemplified detect the activity of an agent on a wide variety of biological mechanisms relevant to fibrosis.

Agents identified by the methods of the invention can serve as the active ingredient in pharmaceutical compositions formulated for the treatment of various fibrotic disorders.

### DATA ANALYSIS

The data from a typical "system", as used herein, provides a single cell type or combination of cell types (where there are multiple cells present in a well) in an *in vitro* culture condition. Primary cells are preferred, or in the case of macrophages, cells derived from primary cells, to avoid potential artifacts introduced by cell lines. In a system, the culture conditions provide a common biologically relevant context. Each system comprises a control, e.g. the cells in the absence of the candidate biologically active agent, although usually the control includes the factors that are present in the test culture. The samples in a system are usually provided in triplicate, and may comprise one, two, three or more triplicate sets.

As used herein, the biological context refers to the exogenous factors added to the culture, which factors stimulate pathways in the cells. Numerous factors are known that induce pathways in responsive cells. By using a combination of factors to provoke a cellular response, one can investigate multiple individual cellular physiological pathways and simulate the physiological response to a change in environment.

As used herein, the term "fibrosis context system" refers the assays HDF-3CGF; HDF-TNFTGF; HDF-3C(-GF); BE4T; BF4T, and M as defined below. Biological contexts of interest for fibrosis include fibroblast cells from any one of a variety of tissues, e.g. dermal, lung, solid tissues, etc. Candidate agents may be tested in one, two, three, four or more assays selected from HDF-3CGF; HDF-TNFTGF; HDF-3C(-GF); BE4T; BF4T, and M. Candidate agents may also be tested in additional assays, for example as set forth in Table 1 below.

**Table 1.**

| BioMAP Systems used to screen for anti-fibrotic agents with desired features. | | | |
|---|---|---|---|
| System | Cell Types | Environment | Readout Parameters |
| 3C | Umbilical Vein Endothelial Cells | IL-1β+TNF-α+IFN-γ | MCP-1, VCAM-1, E-selectin, uPAR, HLA, SRB, Proliferation |
| 4H | Umbilical Vein Endothelial Cells | IL-4+histamine | MCP-1, Eotaxin-3, VCAM-1, uPAR, SRB, VEGFR2 |
| LPS | Peripheral Blood Mononuclear Cells + Umbilical Vein Endothelial Cells | TLR4 | VCAM-1, TF, CD40, E-selectin, CD69, IL-8, M-CSF, PGE2, TNF-α, SRB |
| SAg | Peripheral Blood Mononuclear Cells + Umbilical Vein Endothelial Cells | Superantigens | MCP-1, CD38, CD40, E-selectin, CD69, II-8, MIG, PBMC Cytotoxicity, Proliferation, SRB |
| SM3C | Umbilical Artery Smooth Muscle Cells | IL-1β+TNF-α+IFN-γ | MCP-1, TM, TF, IP-10, IL-8, IL-6, M-CSF, Proliferation, SRB, SAA |
| HDF3CT | Fibroblasts | IL-1β+TNF-α+IFN-γ+TGF-β | IP10, collagen I, MMP-1, SRB |
| BF4T | Bronchial epithelial cells + Fibroblasts | IL-4+TNF-α | MCP-1, Eotaxin-3, VCCAM-1, ICAM-1, IL-8, II-1a, MMP-1, MMP-3, MM-9, PAI-1, SRB, TGF-b, tPA, uPA |
| BE3C | Bronchial epithelial cells | IL-1β+TNF-α+IFN-γ | ICAM-1, UPAR, IP-10, I-TAC, IL-8, MIG, EFR, HLA-DR, II-1a, MMP-1, MMP-9, PAI-1, SRB, TGF-b, tPA, uPA |
| BE4T | Bronchial epithelial cells | IL-4+TNF-a | Eotaxin-3, ICAM-1, IL-8, Collagen I, EGFR, IL-1a, MMP-9, PAI-1, SRB, TGF-b, tPA, uPA |
| HDF3CGF | Fibroblasts | IL-1β+TNF-α+ IFN-γ+EGF+bFGF+ PDGFbb | MCP-1, VCAM-1, CAM-1, IP-10, I-TAC, IL-8, MIG, collagen I, Collagen III, EGFR, M-CSF, MMP-1, PAI-1, Proliferation, SRB, TGF-b, TIMP-1, TIMP-2 |
| HDFT | Fibroblasts | TGF-β | Collagen 1, Collagen III, PAI-1, SRB, bFGF |

| | | | |
|---|---|---|---|
| SRB, sulforhodamine B | | | |

Factors are used in the cultures at conventional concentrations that are sufficient to activate pathways in responsive cells, for example at a concentration of from about 0.1 ng/ml to about 100 ng/ml. Exemplary concentrations include TNF (5 ng/ml), IL-1 (1 ng/ml), IFNγ (10-20 ng/ml), EGF (10 nM), bFGF (10 nM), PDGFbb (10 nM); human epidermal growth factor 10 ng/m; TGFβ (20 ng/ml); M-CSF (50 ng/ml); IL-4 (20 ng/ml); IL-13 (20 ng/ml), IL-6 (20 ng/ml), or GM-CSF (10 ng/ml)

In the "HDF-3CGF" system, dermal fibroblast cells, usually primary human dermal fibroblast cells are cultured alone or in the presence of lung epithelial cells, usually primary human lung epithelial cells, with two or more factors selected from: TNF, IL-1, IFNγ, EGF, bFGF+HSPG (heparan sulfate proteoglycan), and PDGFbb. Three or more factors may be present, four or more, five or more, or all six factors may be present.

In the "HDF-TNFTGF" system, dermal fibroblast cells, usually primary human dermal fibroblast cells are cultured alone or in the presence of lung epithelial cells, usually primary human lung epithelial cells, with two or more of the factors selected from: TGFβ, TNF-α, IL-4 and IGF2. In Three factors may be present.

In the "HDF-3C(-GF)" system, dermal fibroblast cells, usually primary human dermal fibroblast cells, are cultured alone or in the presence of lung epithelial cells, usually primary human lung epithelial cells, with two or more of the factors selected from: IL-1β, TNF-α and IFN-γ. Three or more factors may be present.

Useful parameters for any one of the HDF-TNFTGF; HDF-3CGF or HDF-3C(-GF) systems include, without limitation, ICAM, VCAM, CD40, CD90, IP-10, MCP-1, Collagen I, Mig, m-CSF, TIMP-2, PAI-1, IL-8, Collagen III, HLA-DR, MMP-1, MMP-9, proliferation, TGF-b1, eotaxin-3, decorin, alpha-SMC, MLCK, I-TAC, EGFR, and TIMP-1.

In the "BE4T" system, bronchial epithelial cells, usually primary human bronchial epithelial cells, are cultured in the presence of TNF-α and IL-4.

In the "BF4T" system, bronchial epithelial cells, usually primary human bronchial epithelial cells, are cultured with fibroblasts, usually primary human fibroblasts, in the presence of TNF-α and IL-4. In addition to the factors, stimuli of interest that may be included in the culture medium include TGF-β, IL-5, II-10, IL-9, Tryptase, GM-CSF, II-17, CD40L, Histamine, IgE stimulated Mast Cells, LTB4 stimulated neutrophils, and PBMC stimulated with LPS or SAg.

Useful parameters for either of BE4T or BF4T include, without limitation, CD90, Keratin 8/18, Eotaxin-3, I-TAC, ICAM-1, EGFR, IL-1α, IL-8, MCP-1, MMP-9, MMP-1, MMP-3, PAI-1, TGF-β1, TIMP-2, uPA, tPA, CD87 and VCAM-1. Additional readouts of interest may include IP-10, Elafin/SKALP, Endothelin-1, Gro-a, CD119, IL-6, GM-CSF, IL-16, FGF, PDGF, CD44, E-cadherin, CD40, IL-15Rα, CD1d, CD80, CD86, TARC, eotaxin-1, CD95, MCP-4 and MIP-3a.

In the "M" system, monocytes, including so-called M2 macrophages, may be used. Monocyte sources of interest include freshly isolated human peripheral blood mononuclear cells. Monocytes may be added to confluent monolayers of dermal or adult lung fibroblasts, which are then cultured with two or more factors selected from: TGF-β1; M-CSF; apoptotic bronchial epithelial cells (1:1 ratio with monocytes), IL-4; IL-13; IL-6; IFN-γ; and GM-CSF.

Useful parameters include, without limitation, TGF-β1, mannose receptor, CD23, CD36, CD68, HLADR, DC-SIGN, CR1, annexin-1, SAA, CD1a, cystatin C, FLIP, ADAM15, CD16, CD64, LIGHT, I-309, CD14, CD40, CD69, CD86, CD80, CD163, CD13, E-Selectin, TNF-alpha, IL-1alpha, IL-1beta, IL-6, IL-8, IL-10, IL-12, IL-18, M-CSF, MIP-1a, MIP-3alpha, Mac-1 (CD11b/CD18), MCP-1, MCP-4, fibronectin, MDC, MIG, MMP9, MMP13, urokinase-type plasminogen activator receptor (uPAR, CD87), tissue factor (CD142), transferrin and VCAM-1 (CD106).

The present invention can be applied to the identification of compounds that inhibit or stabilize fibrotic responses. Such compounds have utility in the treatment of fibrosis related disorders.

Results for specific parameters indicate that an agent has desirable features for treatment of fibrosis, e.g. as set forth in Table 2. Compounds that provide for a predetermined number of desired changes are identified as suitable for development in the treatment of fibrosis, e.g. a compound may match the desired change for 10 or more, 20 or more, 30 or more, 40 or more, or 50 or more parameters, where one parameter change in one assay is scored as a change. Some parameters may be additionally scored as a negative if there is not a match with the desired change. Parameters where a failure to correlate with the desired result may be counted against the total score of a candidate agent include those set forth in Table 3, i.e. IL-8 (BE3C, BF4T, BE4T); MIG (SAg); IP-10 (BE3C, HDF3CGF); HLA-DR (3C); uPAR (3C, 4H); MCP-1 (4H, others); TNF-a (LPS); PGE2 (LPS); E-selectin (SAg); IL-6 (SM3C); Eotaxin-3 (4H); MMP-1 (BF4T, HDF3CGF); MMP-3 (BF4T); MMP-9 (BF4T, BE3C); TF (3C); TM (3C, LPS); IL-1a (BE3C, BF4T, BE4T); PAI-1 (various); TGF-betal (various); EGFR (BE4T, BE3C, HDF3CGF); Collagen I (BE4T, HDFT, HDF3CGF); Collagen III (HDFT, HDF3CGF); tPA (BF4T, BE3C); Proliferation (HDF3CGF); uPA (BF4T); TIMP-1 (HDFT, HDF3CGF); M-CSF (LPS); and TIMP-2 (HDFT, HDF3CGF).

**Table 2.**

| Desired biological activities of an anti-fibrotic drug [parameter (system)]. | | |
|---|---|---|
| **Parameter** | **Desired Change** | **Biological Rationale** |
| IL-8 (3C) | increase | indicative of TGF-b pathway inhibition |
| IL-8 (BE3C, BF4T, BE4T) | decrease | increase associated with squamous differentiation |
| MIG (SAg) | no change | Indicative of IFNγ production from T cells |
| IP-10 (BE3C, HDF3CGF) | no change | indicative of IFN-γ signaling and prevention of angiogenesis |
| HLA-DR (3C) | decrease | reduce T cell activation |
| uPAR (3C, 4H) | increase | promotes uPA activity and matrix degredation |
| MCP-1 (4H, others) | decrease | reduces monocyte infiltration and indicative of reduced oxidative stress |
| TNF-α (LPS) | decrease | reduced inflammation and TGF-b production |
| PGE2 (LPS) | increase | antagonizes TGF-β pathway |
| E-selectin (SAg) | decrease | indicative of reduced TNF-a production by T cells |
| IL-6 (SM3C) | decrease | reduces the profibrotic environment |
| Eotaxin-3 (4H) | decrease | indicative of reduced IL-4/13 signaling |
| MMP-1 (BF4T, HDF3CGF) | increase | beneficial maxtrix remodeling as in normal wound healing |
| MMP-3 (BF4T) | decrease | association with fibrotic envents |
| MMP-9 (BF4T, BE3C) | decrease | inhibit pathological matrix remodeling |
| TF (3C) | decrease | reduce fibrin deposition |
| TM (3C, LPS) | increase | inhibit fibrin deposition |
| IL-1a (BE3C, BF4T, BE4T) | decrease | indicative of epithelial wounding |
| PAI-1 (various) | decrease | indicative of TGF-β pathway inhibition and augment fibronolysis while promoting epithelial migration |
| TGF-betal | decrease | drives TGF-β pathway |
| EGFR (BE4T, BE3C, HDF3CGF) | no change or increase | mild blockade of EGFR reduces mucous secretion and MMP production |
| Collagen I (BE4T, HDFT, HDF3CGF) | decrease | reduce collagen deposition |
| Collagen III (HDFT, HDF3CGF) | no change or decrease | reduce provisional matrix deposition that promotes myofibroblast formation and contraction |
| tPA (BF4T, BE3C) | increase | promote provisional fibrin matrix breakdown |
| Proliferation (HDF3CGF) | decrease | reduce fibroblast foci |
| uPA (BF4T) | increase | promote cell migration and remodeling |
| TIMP-1 (HDFT, HDF3CGF) | decrease | promote matrix breakdown |
| M-CSF (LPS) | decrease | promotes M2 macrophage formation |
| TIMP-2 (HDFT, HDF3CGF) | decrease | promote matrix breakdown |
| *EC, endothelial cell (umbilical vein); SMC, smooth muscle cell (coronary artery), monocytes, peripheral blood monocyte; HDF, dermal fibroblast, BrEPI, bronchial epithelial. | | |

**Table 3**

| Parameters where a failure to correlate with the desired result may be counted against the total score of a candidate agent | |
|---|---|
| Parameter | Fibrosis Context Assay |
| IL-8 | BE3C, BF4T, BE4T |
| MIG | SAg |
| IP-10 | BE3C, HDF3CGF |
| HLA-DR | 3C |
| uPAR | 3C, 4H |
| MCP-1 | 4H, others |
| TNF-a | LPS |
| PGE2 | LPS |
| E-selectin | SAg |
| IL-6 | SM3C |
| Eotaxin-3 | 4H |
| MMP-1 | BF4T, HDF3CGF |
| MMP-3 | BF4T |
| MMP-9 | BF4T, BE3C |
| TF | 3C |
| TM | 3C, LPS |
| IL-1a | BE3C, BF4T, BE4T |
| PAI-1 | any |
| TGF-betal | any |
| EGFR | BE4T, BE3C, HDF3CGF |
| Collagen I | BE4T, HDFT, HDF3CGF |
| Collagen III | HDFT, HDF3CGF |
| tPA | BF4T, BE3C |
| Proliferation | HDF3CGF |
| uPA | BF4T |
| TIMP-1 | HDFT, HDF3CGF |
| M-CSF | LPS |
| TIMP-2 | HDFT, HDF3CGF |

A biomap dataset comprises values obtained by measuring parameters or markers of the cells in a system. Each dataset will therefore comprise parameter output from a defined cell type(s) and biological context, and will include a system control. As described above, each sample, e.g. candidate agent, genetic construct, etc., will generally have triplicate data points; and may be multiple triplicate sets. Datasets from multiple systems may be concatenated to enhance sensitivity, as relationships in pathways are strongly context-dependent. It is found that concatenating multiple datasets by simultaneous analysis of 2, 3, 4 or more of the systems as defined herein will provide for enhanced sensitivity of the analysis.

By referring to a biomap is intended that the dataset will comprise values of the levels of at least three sets of parameters, more preferably 4 parameters, and may comprise five, six or more parameters.

The parameters may be optimized by obtaining a system dataset, and using pattern recognition algorithms and statistical analyses to compare and contrast different parameter sets. Parameters are selected that provide a dataset that discriminates between changes in the environment of the cell culture known to have different modes of action, *i.e.* the biomap is similar for agents with a common mode of action, and different for agents with a different mode of action. The optimization process allows the identification and selection of a minimal set of parameters, each of which provides a robust readout, and that together provide a biomap that enables discrimination of different modes of action of stimuli or agents. The iterative process focuses on optimizing the assay combinations and readout parameters to maximize efficiency and the number of signaling pathways and/or functionally different cell states produced in the assay configurations that can be identified and distinguished, while at the same time minimizing the number of parameters or assay combinations required for such discrimination. Optimal parameters are robust and reproducible and selected by their regulation by individual factors and combinations of factors.

Parameters are quantifiable components of cells. A parameter can be any cell component or cell product including cell surface determinant, receptor, protein or conformational or posttranslational modification thereof, lipid, carbohydrate, organic or inorganic molecule, nucleic acid, e.g. mRNA, DNA, *etc.* or a portion derived from such a cell component or combinations thereof. While most parameters will provide a quantitative readout, in some instances a semi-quantitative or qualitative result will be acceptable. Readouts may include a single determined value, or may include mean, median value or the variance, *etc.*

Markers are selected to serve as parameters based on the following criteria, where any parameter need not have all of the criteria: the parameter is modulated in the physiological condition that one is simulating with the assay combination; the parameter has a robust response that can be easily detected and differentiated; the parameter is not co-regulated with another parameter, so as to be redundant in the information provided; and in some instances, changes in the parameter are indicative of toxicity leading to cell death. The set of parameters selected is sufficiently large to allow distinction between datasets, while sufficiently selective to fulfill computational requirements.

Parameters of interest include detection of cytoplasmic, cell surface or secreted biomolecules, frequently biopolymers, e.g. polypeptides, polysaccharides, polynucleotides, lipids, *etc.* Cell surface and secreted molecules are a preferred parameter type as these mediate cell communication and cell effector responses and can be readily assayed. In one embodiment, parameters include specific epitopes. Epitopes are frequently identified using specific monoclonal antibodies or receptor probes. In some cases the molecular entities comprising the epitope are from two or more substances and comprise a defined structure; examples include combinatorially determined epitopes associated with heterodimeric integrins. A parameter may be detection of a specifically modified protein or oligosaccharide, *e.g.* a phosphorylated protein, such as a STAT transcriptional protein; or sulfated oligosaccharide, or such as the carbohydrate structure Sialyl Lewis x, a selectin ligand. The presence of the active conformation of a receptor may comprise one parameter while an inactive conformation of a receptor may comprise another, e.g. the active and inactive forms of heterodimeric integrin α_{M}β₂ or Mac-1.

The treatment options for fibrosis related conditions at present are very limited, although research trials using different drugs that may reduce fibrous scarring are ongoing, and some types of lung fibrosis respond to immunosuppressants and anti-inflammatory drugs.

The term "genetic agent" refers to polynucleotides and analogs thereof, which agents are tested in the screening assays of the invention by addition of the genetic agent to a cell. Genetic agents may be used as a factor, e.g. where the agent provides for expression of a factor. Genetic agents may also be screened, in a manner analogous to chemical agents. The introduction of the genetic agent results in an alteration of the total genetic composition of the cell. Genetic agents such as DNA can result in an experimentally introduced change in the genome of a cell, generally through the integration of the sequence into a chromosome. Genetic changes can also be transient, where the exogenous sequence is not integrated but is maintained as an episomal agents. Genetic agents, such as antisense oligonucleotides, can also affect the expression of proteins without changing the cell's genotype, by interfering with the transcription or translation of mRNA. The effect of a genetic agent is to increase or decrease expression of one or more gene products in the cell.

Agents are screened for biological activity by adding the agent to cells in the system; and may be added to cells in multiple systems. The change in parameter readout in response to the agent is measured to provide the biomap dataset.

The data, particularly data from multiple fibrosis are analyzed by a multiparameter pattern recognition algorithm. For example, the data may be subjected to non-supervised hierarchical clustering to reveal relationships among profiles. Hierarchical clustering may be performed, where the Pearson correlation is employed as the clustering metric. Clustering of the correlation matrix, *e.g.* using multidimensional scaling, enhances the visualization of functional homology similarities and dissimilarities. Multidimensional scaling (MDS) can be applied in one, two or three dimensions. Application of MDS produces a unique ordering for the agents, based on the distance of the agent profiles on a line. To allow objective evaluation of the significance of all relationships between compound activities, profile data from all multiple systems may be concatenated; and the multi-system data compared to each other by pairwise Pearson correlation. The relationships implied by these correlations may then be visualized by using multidimensional scaling to represent them in two or three dimensions.

Biological datasets are analyzed to determine statistically significant matches between datasets, usually between test datasets and control, or profile datasets. Comparisons may be made between two or more datasets with multiparameter analysis algorithms, where a typical dataset comprises readouts from multiple cellular parameters resulting from exposure of cells to biological factors in the absence or presence of a candidate agent, where the agent may be a genetic agent, e.g. expressed coding sequence; or a chemical agent, *e.g.* drug candidate.

A prediction envelope is generated from the repeats of the control profiles; which prediction envelope provides upper and lower limits for experimental variation in parameter values. The prediction envelope(s) may be stored in a computer database for retrieval by a user, *e.g.* in a comparison with a test dataset.

The analysis methods described herein may be used in the determination of functional homology between two agents, where all of the parameters are simultaneously compared. As used herein, the term "functional homology" refers to determination of a similarity of function between two candidate agents, e.g. where the agents act on the same target protein, or affect the same pathway. Functional homology may also distinguish compounds by the effect on secondary pathways, *i.e.* side effects. In this manner, compounds or genes that are structurally dissimilar may be related with respect to their physiological function. Parallel analyses allow identification of compounds with statistically similar functions across systems tested, demonstrating related pathway or molecular targets. Multi-system analysis can also reveal similarity of functional responses induced by mechanistically distinct drugs.

### EXAMPLES

The following examples are put forth so as to provide those of ordinary skill in the art with a complete disclosure and description of how to make and use the subject invention, and are not intended to limit the scope of what is regarded as the invention. Efforts have been made to insure accuracy with respect to the numbers used (*e.g.* amounts, temperature, concentrations, etc.) but some experimental errors and deviations should be allowed for. Unless otherwise indicated, parts are parts by weight, molecular weight is weight average molecular weight, temperature is in degrees centigrade; and pressure is at or near atmospheric.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the scope of the appended claims. It is particularly to be understood that the present invention is not limited to the particular embodiments described herein. For example, the invention is not restricted to the particular methodology, protocols, cell lines, animal species or genera, constructs and reagents described herein as such may vary. The foregoing has been merely a description of certain preferred embodiments of the invention, not intended to limit the scope of that invention, which is defined only by the appended claims.

### EXAMPLE 1

### REGULATORS OF FIBROBLAST RESPONSES HDF-IL-1B/TNF-A/IFN-G/EGF/BFGF/PDGFBB, HDF-TGF/TNF-A

The present invention is applied for the screening of compounds that inhibit fibroblast responses.

Human neonatal dermal fibroblasts (HDFn) or adult lung fibroblasts (HDFp) or fibroblasts from tissues such as liver, heart, or kidney are used. Cells are cultured at 3 x 10⁴ cells/ml in DMEM/F12 (50/50) from Cellgro, supplemented with LSGS kit (from Cascade Biologics); fetal bovine serum, 2% v/v, hydrocortisone 100 nM, human epidermal growth factor 10 ng/ml, basic fibroblast growth factor 3 ng/ml and heparin 10 µg/ml, and penicillin/streptomycin amphotericin B solution (PSA), until confluency. Medium is replaced with DMEM/F12 with only penicillin/streptomycin amphotericin B solution (PSA), then 24 hours later, the following are applied:

| **Factors** | **Designation** |
|---|---|
| TNF (5ng/ml), IL-1 (1ng/ml), IFN (20ng/ml), EGF (10 nM), bFGF+HSPG (10 nM+x ug/ml), PDGFbb (10 nM) | HDF-3CGF |
| TGFβ (20ng/ml), TNF (5ng/ml) | HDF-TNFTGF |

After another 24, 48 or 72 hours incubation (37°C, 5% CO₂) the cultures are evaluated for the following parameters: ICAM, VCAM, CD40, CD90, IP-10, MCP-1, Collagen I, Mig, m-CSF, TIMP-2, PAI-I, IL-8, Collagen III, HLA-DR, MMP-1, MMP-9, proliferation, TGF-b1, eotaxin-3, decorin, alpha-SMC, MLCK, I-TAC, EGFR, TIMP-1.

### EXAMPLE 2

### REGULATORS OF FIBROBLAST/BRONCHIAL EPITHELIAL CELL RESPONSES BREPI/HDFN-TNF-α/IL-4

This example describes the screening of compounds that inhibit fibroblast / bronchial epithelial cell responses.

Human neonatal fibroblasts (HDFn) and normal human bronchial epithelial cells (BrEPI) are used. Human lung fibroblasts (adult or neonatal) could also be used. HDFn are cultured in DMEM/F12 (50/50) from Cellgro, supplemented with LSGS kit (from Cascade Biologics); fetal bovine serum, 2% v/v, hydrocortisone 1 µg/ml, human epidermal growth factor 10 ng/ml, basic fibroblast growth factor 3 ng/ml and heparin 10 µg/ml, and penicillin/streptomycin amphotericin B solution (PSA). BrEPI are cultured in supplemented BEBM medium (Cambrex) at 2 x 10⁴/ml. BrEPI may also be cultured with Bronchial/Tracheal Epithelial Cell Serum-Free Growth Medium (Cell Applications, Inc.) or F12/DMEM supplemented with 10 µg/ml rhu-insulin, 10 µg/ml transferrin, 10 ng/ml epidermal growth factor (EGF), 1 µM ethanolamine, 25 µg/ml aprotinin, 25 µg/ml glucose, 1 µM phosphoethanolamine, 5 pM triiodothyronine, 50 nM selenium, 1 nM hydrocortisone, 1 nM progesterone, 5 µM forskolin, 10 µM heregulin ß177-244, 5 µl/ml fibronectin, 4 µl/ml bovine pituitary extract and retinoid acid. HDFn are plated for assays at 3 x 10⁴ cells/ml. After 2 days, media is replaced with BrEPI media containing 10⁴ BrEPI cells and cultured for a further 2 days.

Upon reaching confluency, the test agent or buffer control is added and the following are applied: 5 ng/ml of IL-4 and 5 ng/ml TNF-α (BF4T). After another 24 hours incubation (37°C, 5% CO₂) the cultures are evaluated for the following parameters: CD90, Keratin 8/18, Eotaxin-3, I-TAC, ICAM-1, EGFR, IL-1α, IL-8, MCP-1, MMP-9, MMP-1, MMP-3, PAI-1, TGF-β1, TIMP-2, uPA, tPA, CD87 and VCAM-1. Additional readouts of interest may include IP-10, Elafin/SKALP, Endothelin-1, Gro-a, CD119, IL-6, GM-CSF, IL-16, FGF, PDGF, CD44, E-cadherin, CD40, IL-15Ralpha, CD1d, CD80, CD86, TARC, eotaxin-1, CD95, MCP-4 and MIP-3a. Other stimuli of interest include: TGFbeta, IL-5, II-10, IL-9, Tryptase, GM-CSF, II-17, CD40L, Histamine, IgE stimulated Mast Cells, LTB4 stimulated neutrophils, and PBMC stimulated with LPS or SAg.

### EXAMPLE 3

### REGULATORS OF MACROPHAGE DIFFERENTIATION AND RESPONSES

This example describes the screening of compounds that inhibit the differentiation into and responses of macrophages.

Macrophages are generated from human peripheral blood mononuclear cells. Human peripheral blood mononuclear cells are isolated from blood by Ficoll-hypaque density gradient centrifugation as described (Ponath, JEM 183:2437, 1996). Monocytes are then isolated by negative selection using the Monocyte Isolation Kit II (Miltenyi Biotec, Germany) MACS beads according to the manufacturer's instructions. Alternatively, 10x10⁸ peripheral blood mononuclear cells/ml are cultured in RPMI containing 10% fetal bovine serum for 3 hours and non-adherent lymphocytes are removed by gentle washing. Monocytes are then added to confluent monolayers of neonatal dermal (HDFn) or adult lung (HDFp) fibroblasts. The following are then applied to the coculture for 7 to 8 days alone or in combination: TGF-β1 (10 ng/ml), M-CSF (50ng/ml), apoptotic bronchial epithelial cells (1:1 ratio with monocytes), IL-4 (20 ng/ml), IL-13 (20 ng/ml), IL-6 (20 ng/ml), IFN-gamma (10 ng/ml), or GM-CSF (10 ng/ml).

After 7-8 days, the cultures can be stimulated with a variety of stimuli: a test agent alone or in combination with LPS (2 ng/ml), Zymosan (10 µg/ml), cell wall preparation from Saccharomyces cerevisiae (Underhill DM, et al., Nature, 401(6755):811-5, 1999). Other stimulants that can be substituted for Zymosan in this system include Toll-like receptor ligands such as Poly(I:C) dsRNA, Oligonucleotides with human CpGs, Loxoribine, Pam3Cys synthetic lipoprotein, peptidoglycans, opsonized fixed heat killed bacteria; or immune complexes such as heat agglutinated IgG, anti IgG/IgG, and IgG coated onto microspheres (Polysciences, Inc.).

Based on the parameters altered by the indicated factors, biomaps are generated for the parameters TGF-β1, mannose receptor, CD23, CD36, CD68, HLADR, DC-SIGN, CR1, annexin-1, SAA, CD1a, cystatin C, FLIP, ADAM15, CD16, CD64, LIGHT, I-309, CD14, CD40, CD69, CD86, CD80, CD163, CD13, E-Selectin, TNF-alpha, IL-1alpha, IL-1beta, IL-6, IL-8, IL-10, IL-12, IL-18, M-CSF, MIP-1a, MIP-3alpha, Mac-1 (CD11b/CD18), MCP-1, MCP-4, fibronectin, MDC, MIG, MMP9, MMP13, urokinase-type plasminogen activator receptor (uPAR, CD87), tissue factor (CD142), transferrin and VCAM-1 (CD106).

### EXAMPLE 4

### SCREENING FOR ANTI-FIBROTIC Agents

The present invention relates to methods to identify agents useful in the treatment of fibrosis, based on the identification of drugs that display a favorable combination of features (or biological activities of importance), defined as (1) inhibition of matrix remodeling with promotion of wound healing, (2) protection of epithelial health and growth, (3) controlling provisional fibrin matrix deposition, and (4) selected anti-inflammatory activities.

The inventive methods and compositions provide a system for the assessment of candidate therapies for fibrotic disease, particularly in epithelial tissues, including pulmonary fibrosis, systemic sclerosis, renal fibrosis, hepatic fibrosis, *etc.*

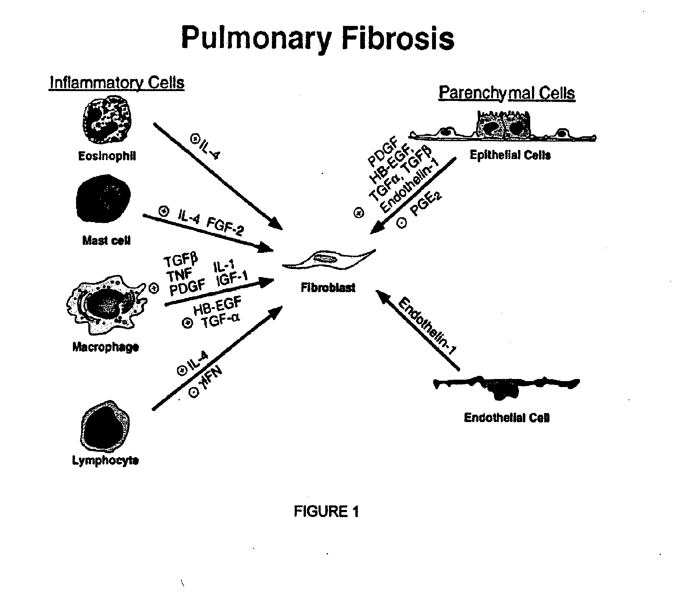

In order to analyze agents that modulate fibrotic processes, particularly those of relevance to disease, model systems containing fibroblasts, e.g. dermal fibroblasts, lung fibroblasts, hepatic fibroblasts, etc., epithelial cells, e.g. bronchial epithelial cells; endothelial cells, macrophages, myofibroblasts *etc*. are used. The multi-cell and/or multifactor design of the systems and their analysis through multi-parameter activity profiles work together to optimize information content, enabling rapid but effective analysis of drug activities in complex cellular responses relevant to clinical disease. The BioMAP Systems that model the relevant biological processes outlined in Figure 1 and are used for screening are listed in Table 1. Such BioMap systems are generally primary human cell based assays. Compounds are screened in one or more such BioMap systems, usually in at least about two such systems, and may be screened in at least three, at least four, at least five, at least ten, and up to all of the assays set forth in Table 1.

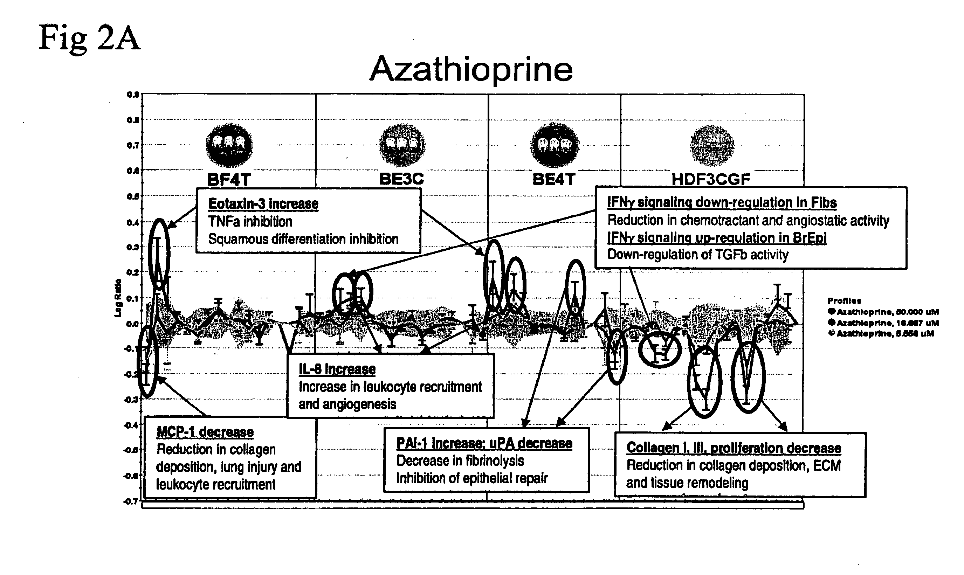

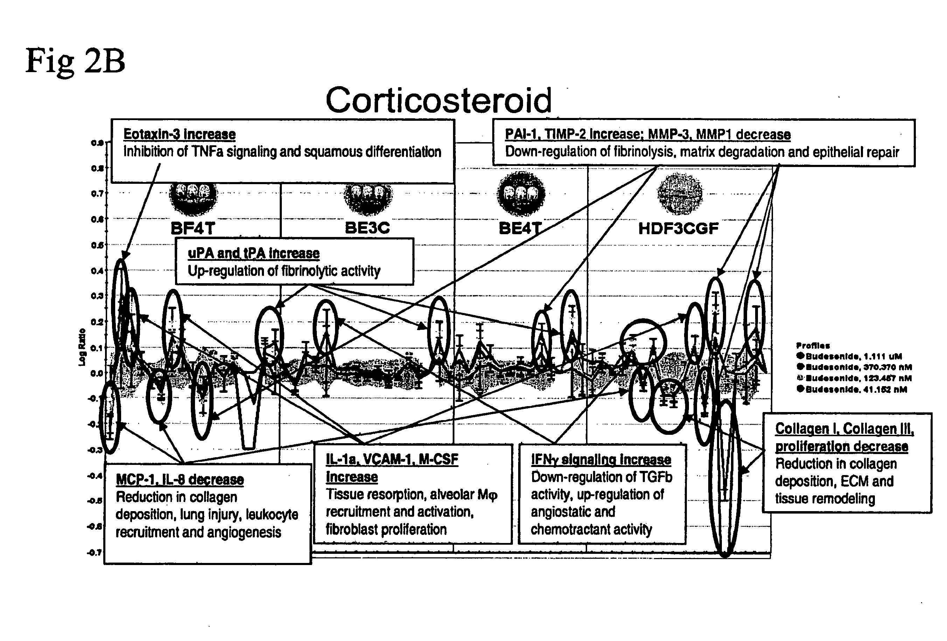

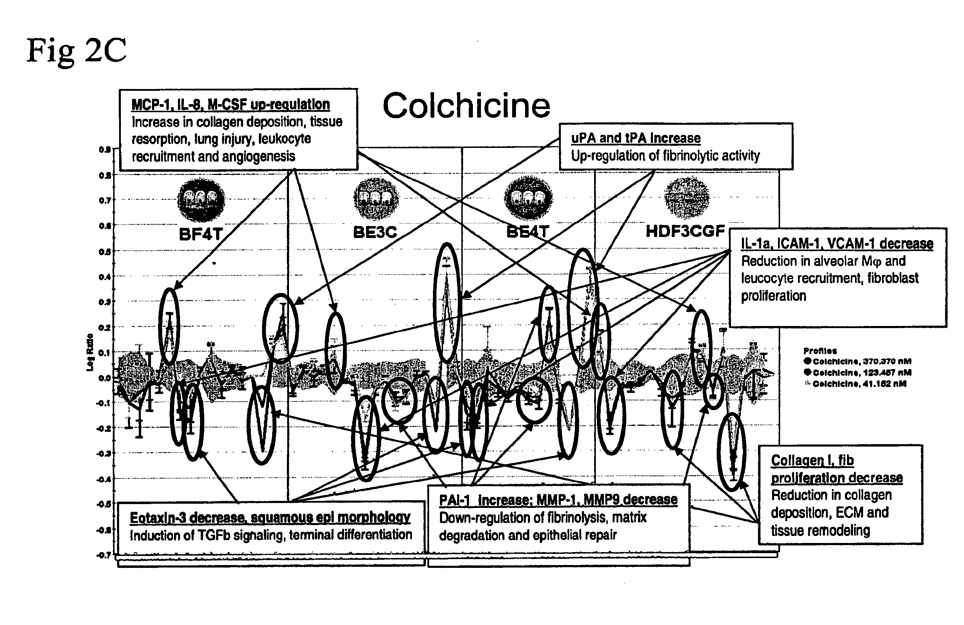

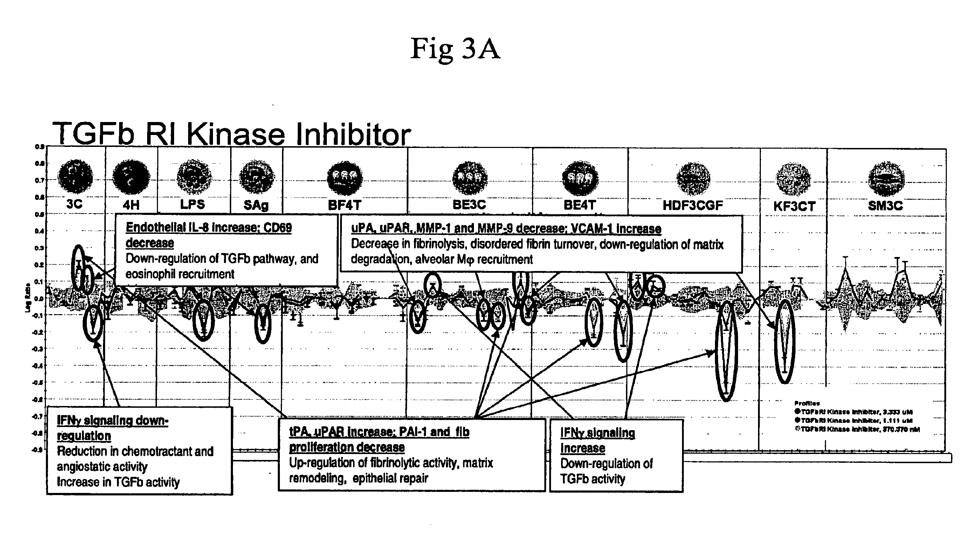

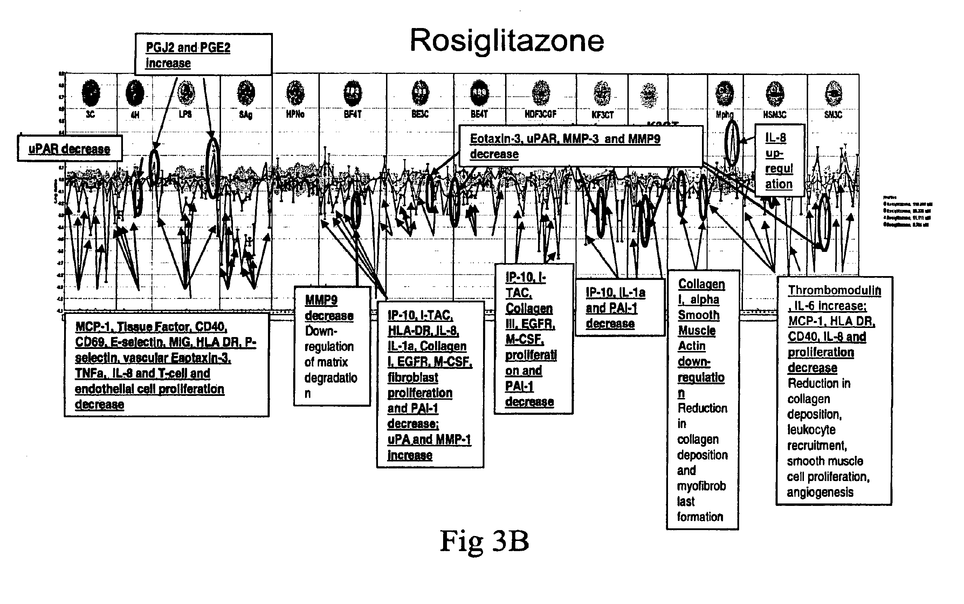

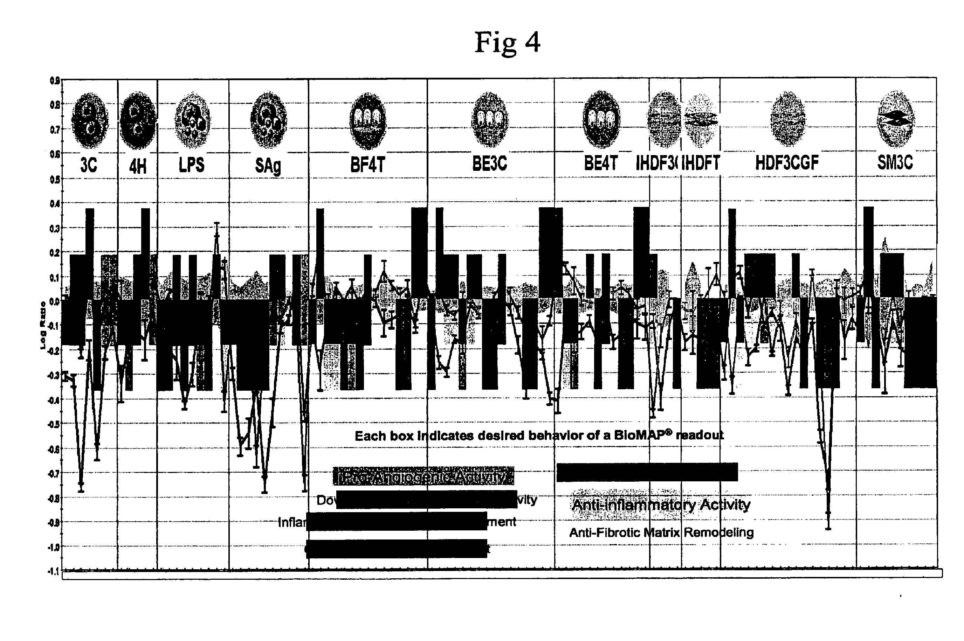

Using the systems described above, relevant drugs actually used to treat fibrosis (e.g. azathioprine, budesonide, and colchicine) and experimental drugs purported to be efficacious (e.g. rosiglitazone and TGF-b kinase inhibitor) were tested in the fibrosis systems as shown in Figures 2A, 2B, 2C, and 3A and 3B. Based on the activities of these drugs, as shown in Figure 4 and Table 2, various parameters were determined to be desirable for an effective anti-fibrotic agent. For instance, inhibition of the TGF-b pathway appears to promote the health of epithelial cells and a reduction in MMP production, so an anti-fibrotic agent should have some level of TGF-b inhibition as indicated by e.g. MMP-9 inhibition or IL-8 upregulation. Similarly, rosiglitazone, a PPARg ligand seems to have anti-fibrotic activity, upregulates PGE2, which is known to antagonize TGF-b activity, making PGE2 upregulation a desired feature. The features in Table 2 were chosen in a similar manner.

Description of the biological rationale, and the corresponding markers (or readout parameters), which are used to read out the rationale are listed in Table 2. A suitable anti-fibrotic agent will modulate the parameters in the given system in the desired direction, e.g. a desired anti-fibrotic will upregulate IL-8 in the 3C system which indicates inhibition of the TGF-β pathway.

Each screened compound is scored according to its modulation of the selected readout parameters. Compounds receive a positive score (score of 1) or negative score (score of 0) for each readout parameter set forth in Table 2 that is modulated in the desired direction. The compounds that score higher than current therapeutics (e.g. prednisolone or colchicine) are considered an improvement. Parameters may be penalized by making the positive score a -1 for parameters where regulation is opposite to the desired direction. That is, if a compound downregulated IL-8 in the 3C system, it may be scored a -1 instead of a just a 0.

The relative importance of each parameter may be weighted by the screener to select those parameters of greatest interest for a particular application. While a preferred drug modulates all of the readout parameters in the desired way; desirable bioactive compounds may also modulate only one or several of the parameters in the desired fashion. One can combine two or more drugs such that more of the desired parameter changes are obtained than either drug is capable of inducing alone. For fibrosis, preferred compounds for development in treating fibrosis are those that have high overall score.

A library of over 1100 physiologically active compounds was screened in the assays listed in Table 1, for the activities listed in Table 2. Compounds were first selected to inhibit PAI-1 in the HDFT as an indication of TBF-b signaling inhibition without being cytotoxic. 39 compounds met this inhibition of PAI-1 criterion. Of these 39, 18 compounds had overall scores better than the current therapies (Table 4 and Table 5). Blank spots in Table 3 were where parameter was not assayed. Penalties were not applied for this scoring.

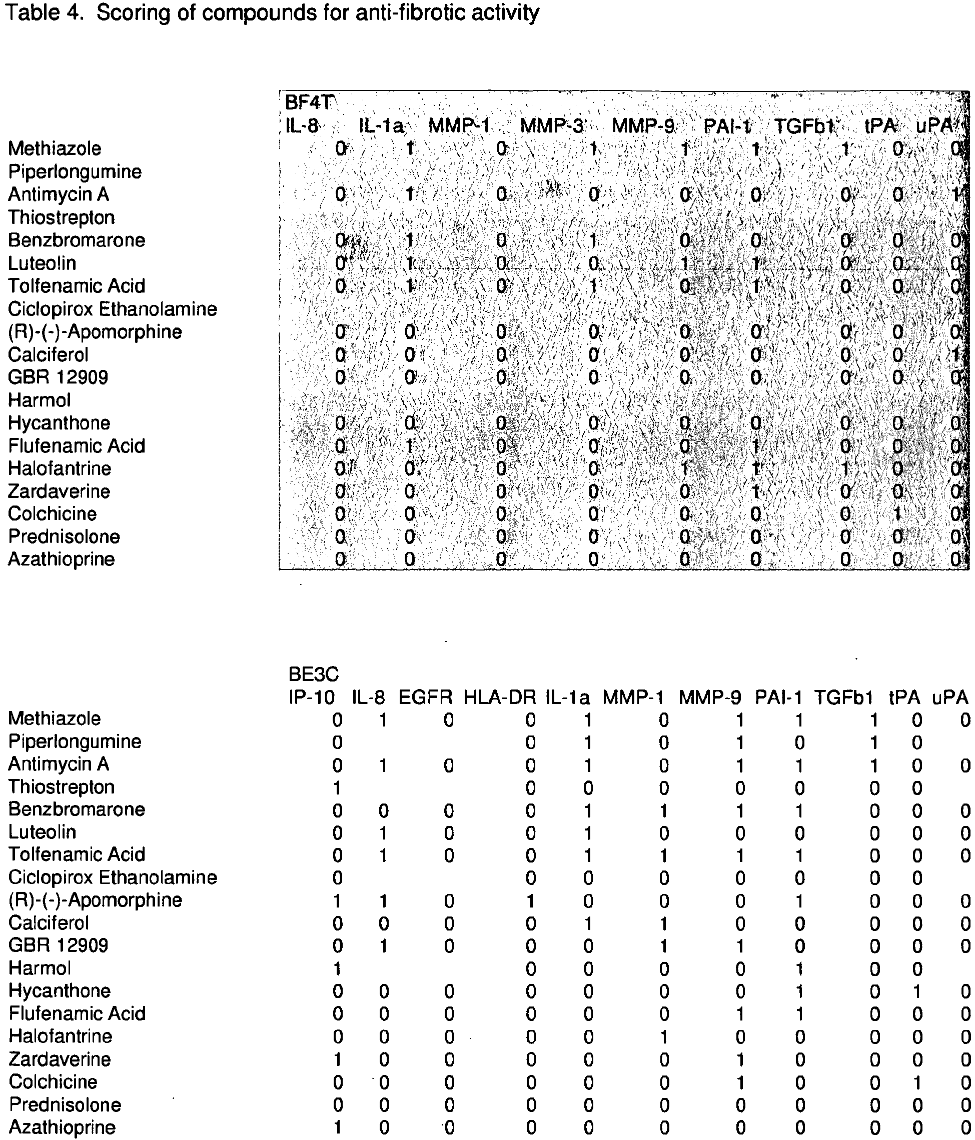

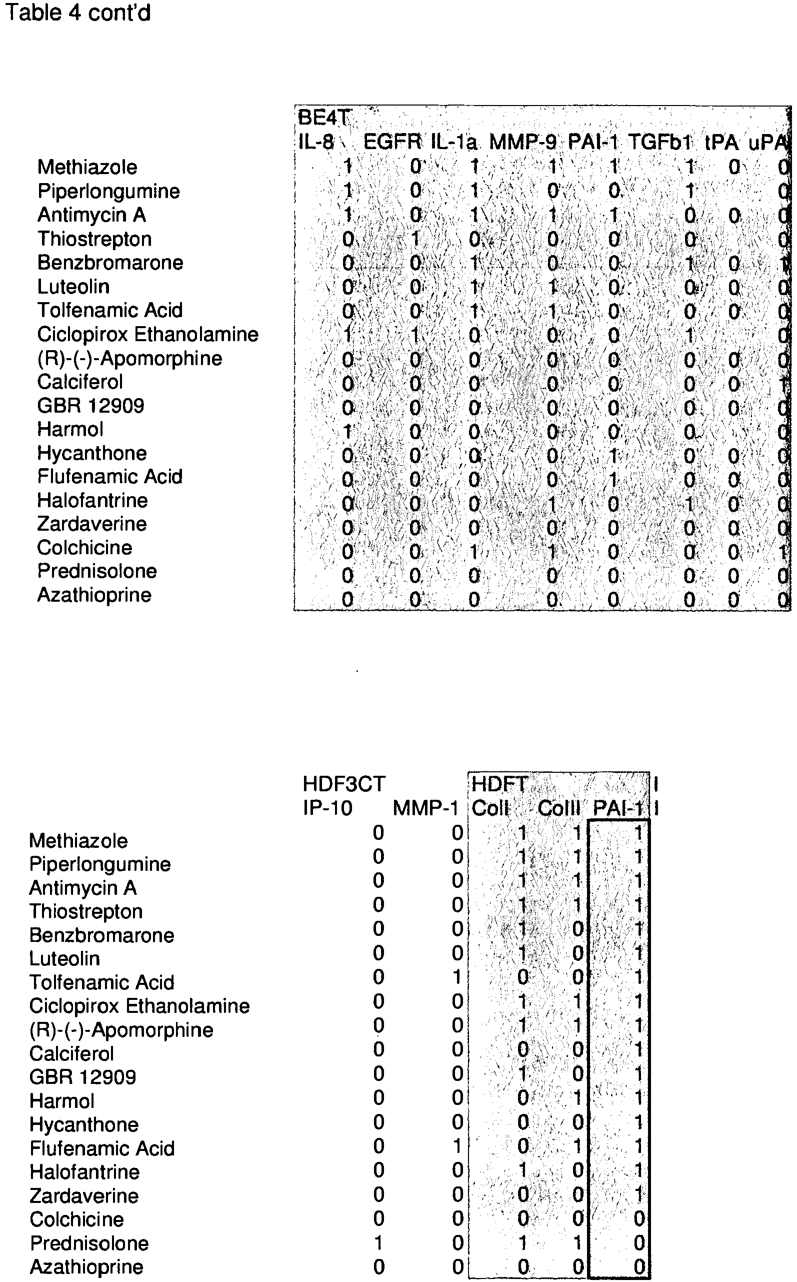

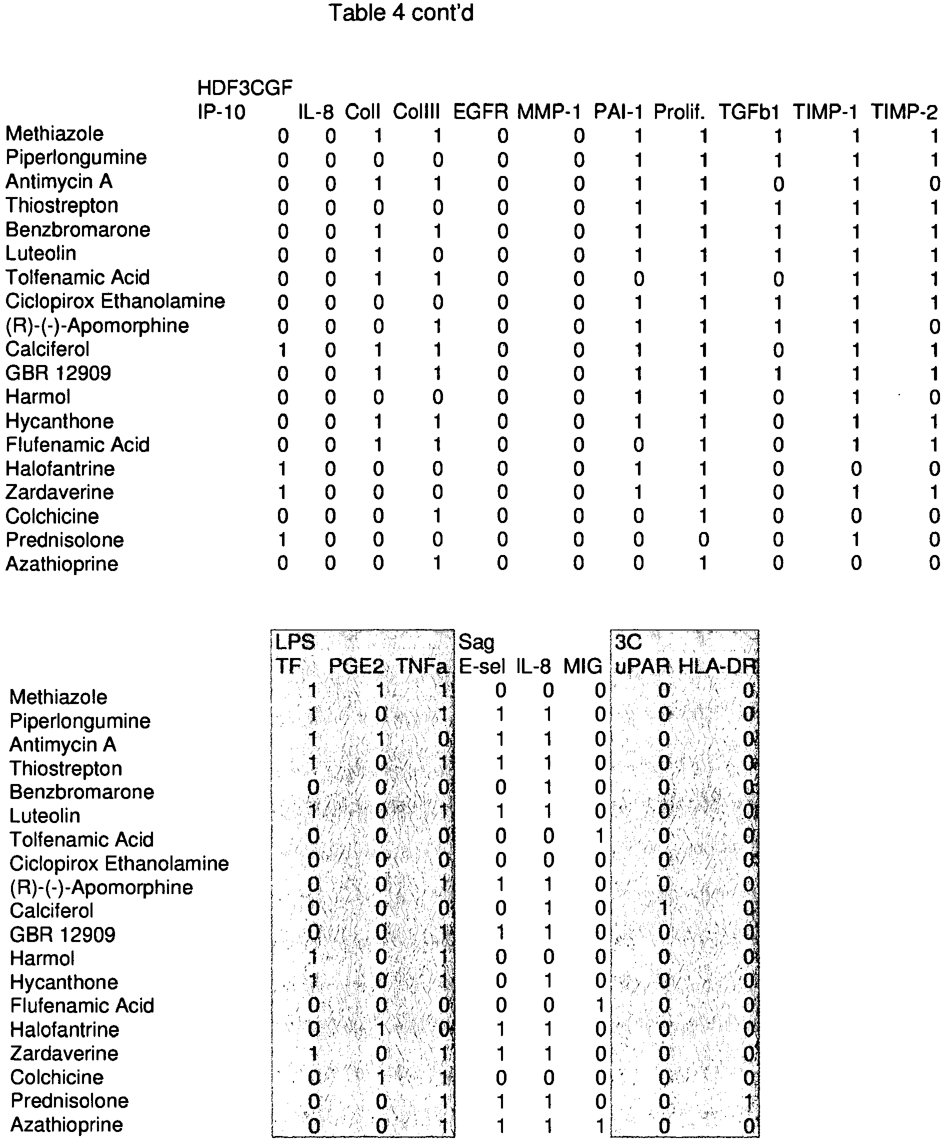

Overall scores of the currently used therapies prednisolone, azathioprine, and colchicine are shown for comparison (however, none of these inhibit PAI-1 in the HDFT system). Thus, compounds that have overall scores equal or higher than these compounds are considered an improvement over the existing drugs for the treatment of fibrosis. Among the compounds with high scores in the BioMAP screen were retinoic acid (PMID 17023731) and spironolactone (PMID 8421998), compounds with known anti-fibrotic activity, thus confirming the validity of the screening method described here.

**Table 5. Total score for desired parameter modulation.**

| Drug | Score (Fraction correctly modulated parameters out of 45-58 total) |
|---|---|
| Methiazole | 0.55 |
| Piperlongumine | 0.47 |
| Antimycin A | 0.45 |
| Thiostrepton | 0.42 |
| Benzbromarone | 0.36 |
| Luteolin | 0.36 |
| Tolfenamic Acid | 0.33 |
| Ciclopirox Ethanolamine | 0.31 |
| (R)-(-)-Apomorphine | 0.31 |
| Calciferol | 0.29 |
| GBR 12909 | 0.29 |
| Harmol | 0.29 |
| Hycanthone | 0.28 |
| Flufenamic Acid | 0.28 |
| Halofantrine | 0.27 |
| Zardaverine | 0.25 |

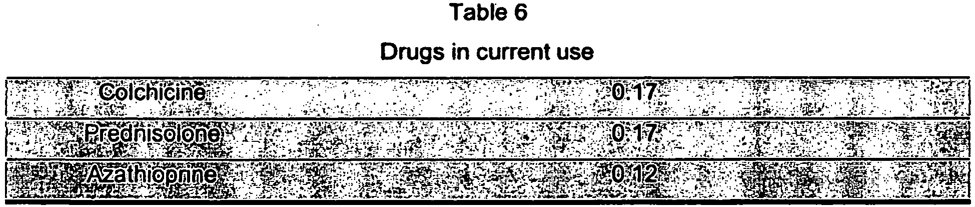

BioMAP Systems are designed to model complex human disease biology in a practical *in vitro* format. This is achieved by stimulating human primary cells (single cell type or defined mixtures of cell types) such that multiple disease-relevant signaling pathways are simultaneously active. The choice of cell types and stimulations is guided by the knowledge of disease biology and mechanisms. Incorporating appropriate cell types and stimulating signaling pathways relevant to disease states allows association of biological activities detected in BioMAP Systems with disease processes. Drug effects are then recorded by measuring biologically meaningful protein readouts that provide coverage of biological space of interest (e.g., inflammation, tissue remodeling) and allow discrimination between different drug classes tested. The BioMAP systems useful for this evaluation are listed in **Table 1.**

Cell culture. Human umbilical vein endothelial cells (HUVEC) were pooled from multiple donors, cultured according to standard methods, and plated into microtiter plates at passage **4.** Cell culture medium for all systems containing HUVEC included hydrocortisone. Coronary artery smooth muscle cells (CASM3C) and umbilical artery smooth muscle cells (HSM3C) were cultured according to standard procedures. Peripheral blood mononuclear cells (PBMC) were prepared from buffy coats from normal human donors according to standard methods. Monocyte-derived macrophages were differentiated in the presence of M-CSF for 7 d according to standard procedures. Human dermal fibroblasts were pooled from 3 donors, cultured according to standard methods, and plated confluent 24 h before stimulation. Cell culture medium for all systems containing fibroblasts and keratinocytes contained low hydrocortisone. Epidermal keratinocytes and bronchial epithelial cells from 3 donors were cultured according to standard procedures and pooled when plated into microtiter plates. For keratinocyte-fibroblast co-cultures, cells were plated simultaneously 24 h before stimulation. For bronchial epithelial cell-fibroblast co-cultures, fibroblasts were plated and cultured 48 before addition of epithelial cells, then cultured another 24 hr before stimulation. Concentrations/amounts of agents added to confluent microtiter plates to build each system: cytokines (IL-1β, 1 ng/ml; TNF-α, 5 ng/ml; IFN-γ, 20 ng/ml; IL-4, 5 ng/ml), activators (SAg, 20 ng/ml; histamine, 10 µM; zymosan, 10 µg/ml; or LPS, 2 ng/ml), growth factors (TGF-β, 5 ng/ml; EGF, bFGF, and PDGF-BB, 10 ng/ml), and PBMC (7.5x10⁴ cells/well) or macrophages (3.5x10⁴ cells/well).

Compounds. Compounds were prepared in the solvent directed, added 1 hr before stimulation of the cells, and were present during the 24 hr stimulation period for the measurement of parameters listed in Table 2. For proliferation, compounds added 1 hr before stimulation of the cells with cytokines, and were present during the 48 to 72 hr assay. If prepared in DMSO, the final concentration of solvent was 0.1% or less.

Readout Parameter Measurements. The levels of readout parameters were measured by ELISA. Briefly, microtiter plates are treated, blocked, and then incubated with primary antibodies or isotype control antibodies (0.01-0.5 microg/ml) for 1 hr. After washing, plates were incubated with a peroxidase-conjugated anti-mouse IgG secondary antibody or a biotin-conjugated anti-mouse IgG antibody for 1 hr followed by streptavidin-HRP for 30 min. Plates were washed and developed with TMB substrate and the absorbance (OD) was read at 450 nm (subtracting the background absorbance at 650 nm). Quantitation of TNF-alpha or PGE2 in the LPS system was done using a commercially available kit according to the manufacturer's directions. Proliferation of endothelial cells (HUVEC and ALEC), smooth muscle cels (SMC and CASMC), and PBMC (T cells) was quantitated by Alamar Blue reduction or SRB.

Toxicity Assessments. Adverse effects of compounds on cells were determined by 1) measuring alterations in total protein (SRB assay), 2) measuring the viability of peripheral blood mononuclear cells (reduction of Alamar blue); and 3) microscopic visualization. SRB was performed by staining cells with 0.1% sulforhodamine B after fixation with 10% TCA, and reading wells at 560 nm. PBMC viability was assessed by adding 10% Alamar blue to PBMC that had been cultured for 16 hours in the presence of activators and measuring the amount of dye reduced over the next 8 h. Samples were assessed visually according to the following scheme: 2.0=cobblestone (unactivated phenotype); 1.0=activated (normal phenotype); 0.5=lacy or sparse; 0.375=rounded; 0.25=sparse and granular; 0.1=no cells in well.

Data analysis. Mean OD values for each parameter were calculated for compounds run in duplicate. The mean value (or single well OD value) for each parameter in a treated sample was then divided by the mean value from an appropriate control to generate a ratio. All ratios were then log₁₀ transformed. 95% significance prediction envelopes (grey shading in Figures) were calculated for historical controls.

## Claims

1. A method for identifying agents that stabilize or reverse fibrosis, the method comprising:
contacting the agent with human dermal fibroblast cells cultured alone or in the presence of lung epithelial cells, with at least five factors selected from TNF, IL-1, IFNγ, EGF, bFGF+HSPG, and PDGFbb acting on the cells;
recording changes in at least three parameters, as parameter readouts, as a result of introduction of the agent, wherein the parameters are selected from ICAM, VCAM, CD40, CD90, IP-10, MCP-1, Collagen I, Mig, m-CSF, TIMP-2, PAI-I, IL-8, Collagen III, HLA-DR, MMP-1, MMP-9, proliferation, TGF-β1, eotaxin-3, decorin, alpha-SMA, MLCK, I-TAC, EGFR, and TIMP-1;
deriving a biological dataset profile from the changes in parameter readouts, where the biological dataset profile has data normalized to be a ratio of test data to control data on the same cell type under control conditions in the absence of the agent; and
analyzing the biological dataset profile by a multiparameter pattern recognition algorithm to quantify relatedness of the biological dataset profile to reference biological dataset profiles that include known agents that target specific pathways, wherein the presence or absence of relatedness to said reference biological dataset profile provides a characterization of the mechanism of action of said agent,
thereby identifying the agent as a potential agent that stabilizes or reverses fibrosis.

2. The method according to Claim 1, wherein the agent is a genetic agent.

3. The method according to Claim 1, wherein the agent is a chemical or biological agent.

4. The method according to Claim 1, wherein at least a second biological dataset profile is concatenated.

5. The method according to Claim 4, wherein at least a third biological dataset profile is concatenated.

6. The method of claim 4, wherein the second biological dataset profile is derived from:
(i) contacting the agent with dermal fibroblast cells cultured alone or in the presence of lung epithelial cells with at least two factors selected from TGFβ, TNFα, IL-4 and IGF2, acting upon cells; and recording changes in at least three different parameters, as parameter readouts, as a result of introduction of the agent, wherein the parameters are selected from ICAM, VCAM, CD40, CD90, IP-10, MCP-1, Collagen I, Mig, m-CSF, TIMP-2, PAI-I, IL-8, Collagen III, HLA-DR, MMP-1, MMP-9, proliferation, TGF-β1, eotaxin-3, decorin, alpha-SMA, MLCK, I-TAC, EGFR, and TIMP-1;
(ii) contacting the agent with dermal fibroblast cells cultured alone or in the presence of lung epithelial cells with at least two factors selected from IL-1β, TNF-α and IFN-γ; and recording changes in at least three different parameters, as parameter readouts, as a result of introduction of the agent, wherein the parameters are selected from ICAM, VCAM, CD40, CD90, IP-10, MCP-1, Collagen I, Mig, m-CSF, TIMP-2, PAI-I, IL-8, Collagen III, HLA-DR, MMP-1, MMP-9, proliferation, TGF-β1, eotaxin-3, decorin, alpha-SMA, MLCK, I-TAC, EGFR, and TIMP-1;
(iii) contacting the agent with bronchial epithelial cells cultured with TNF-α and IL-4; and recording changes in at least three different parameters, as parameter readouts, as a result of introduction of the agent, wherein the parameters are selected from CD90, Keratin 8/18, Eotaxin-3, I-TAC, ICAM-1, EGFR, IL-1α, IL-8, MCP-1, MMP-9, MMP-1, MMP-3, PAI-1, TGF-β1, TIMP-2, uPA, tPA, CD87, VCAM-1, IP-10, Elafin/SKALP, Endothelin-1, Gro-a, CD119, IL-6, GM-CSF, IL-16, FGF, PDGF, CD44, E-cadherin, CD40, IL-15Rα, CD1d, CD80, CD86, TARC, eotaxin-1, CD95, MCP-4 and MIP-3a;
(iv) contacting the agent with bronchial epithelial cells cultured with fibroblasts with TNF-α and IL-4; and recording changes in at least three different parameters, as parameter readouts, as a result of introduction of the agent, wherein the parameters are selected from CD90, Keratin 8/18, Eotaxin-3, I-TAC, ICAM-1, EGFR, IL-1α, IL-8, MCP-1, MMP-9, MMP-1, MMP-3, PAI-1, TGF-β1, TIMP-2, uPA, tPA, CD87, VCAM-1, IP-10, Elafin/SKALP, Endothelin-1, Gro-a, CD119, IL-6, GM-CSF, IL-16, FGF, PDGF, CD44, E-cadherin, CD40, IL-15Rα, CD1d, CD80, CD86, TARC, eotaxin-1, CD95, MCP-4 and MIP-3a; or
(v) contacting the agent with monocytes cultured with fibroblasts in the presence of at least two factors selected from TGF-β1; M-CSF; apoptotic bronchial epithelial cells (1:1 ratio with monocytes), IL-4; IL-13; IL-6; IFN-γ; and GM-CSF and recording changes in at least three different parameters, as parameter readouts, as a result of introduction of the agent, wherein the parameters are selected from TGF-β1, mannose receptor, CD23, CD36, CD68, HLADR, DC-SIGN, CR1, annexin-1, SAA, CD1a, cystatin C, FLIP, ADAM15, CD16, CD64, LIGHT, I-309, CD14, CD40, CD69, CD86, CD80, CD163, CD13, E-Selectin, TNF-alpha, IL-1alpha, IL-1beta, IL-6, IL-8, IL-10, IL-12, IL-18, M-CSF, MIP-1a, MIP-3alpha, Mac-1 (CD11b/CD18), MCP-1, MCP-4, fibronectin, MDC, MIG, MMP9, MMP13, urokinase-type plasminogen activator receptor (uPAR, CD87), tissue factor (CD142), transferrin and VCAM-1 (CD106).

7. The method according to Claim 1, wherein a candidate agent identified as suitable for development in the treatment of fibrosis matches at least 10 desired changes set forth in Table 2.

8. The method according to Claim 6, wherein the total score for determining suitability of a candidate agent for development in the treatment of fibrosis negatively scores a parameter change as set forth in Table 3.

## Patentansprüche

1. Verfahren zur Identifikation von Mitteln, die Fibrose stabilisieren oder umkehren, wobei das Verfahren Folgendes umfasst:
das Kontaktieren des Mittels mit menschlichen dermalen Fibroblastenzellen, die alleine oder in Gegenwart von Lungenepithelzellen mit zumindest fünf Faktoren, die aus TNF, IL-1, IFNγ, EGF, bFGF+HSPG und PDGFbb ausgewählt sind und auf die Zellen wirken, kultiviert wurden;
das Aufzeichnen von Veränderungen in zumindest drei Parametern als Parameterablesungen als Folge der Einbringung des Mittels, wobei die Parameter aus ICAM, VCAM, CD40, CD90, IP-10, MCP-1, Collagen I, Mig, m-CSF, TIMP-2, PAI-I, IL-8, Collagen III, HLA-DR, MMP-1, MMP-9, Proliferation, TGF-β1, Eotaxin-3, Decorin, α-SMA, MCLK, I-TAC, EGFR und TIMP-1 ausgewählt sind;
das Ableiten eines biologischen Datensatzprofils aus den Veränderungen der Parameterablesungen, wobei das biologische Datensatzprofil normalisierte Daten aufweist, die ein Verhältnis zwischen den Testdaten und Kontrolldaten beim gleichen Zelltyp unter kontrollierten Bedingungen in Abwesenheit des Mittels angeben,
das Analysieren des biologischen Datensatzprofils durch einen Multiparametermustererkennungsalgorithmus zur Quantifizierung der Verwandtheit des biologischen Datensatzprofils mit biologischen Bezugsdatensatzprofilen, die bekannte Mittel umfassen, die auf spezifische Stoffwechselwege abzielen, wobei die Gegenwart oder Abwesenheit der Verwandtheit mit dem biologischen Bezugsdatensatzprofil eine Charakterisierung des Wirkmechanismus des Mittels bereitstellt,
wodurch das Mittel als mögliches Mittel zur Stabilisierung oder Umkehr von Fibrose identifiziert wird.

2. Verfahren nach Anspruch 1, wobei das Mittel ein genetisches Mittel ist.

3. Verwendung nach Anspruch 1, wobei das Mittel ein chemisches oder biologisches Mittel ist.

4. Verfahren nach Anspruch 1, wobei zumindest ein zweites biologisches Datensatzprofil verknüpft wird.

5. Verfahren nach Anspruch 4, wobei zumindest ein drittes biologisches Datensatzprofil verknüpft wird.

6. Verfahren nach Anspruch 4, wobei das zweite biologische Datensatzprofil folgendermaßen abgeleitet ist:
(i) Kontaktieren des Mittels mit dermalen Fibroblastenzellen, die alleine oder in Gegenwart von Lungenepithelzellen mit zumindest zwei Faktoren, die aus TGFβ, TNFα, IL-4 und IGF2 ausgewählt sind und auf die Zellen wirken, kultiviert wurden; und das Aufzeichnen von Veränderungen in zumindest drei verschiedenen Parametern als Parameterablesungen als Folge der Einbringung des Mittels, wobei die Parameter aus ICAM, VCAM, CD40, CD90, IP-10, MCP-1, Collagen I, Mig, m-CSF, TIMP-2, PAI-I, IL-8, Collagen III, HLA-DR, MMP-1, MMP-9, Proliferation, TGF-β1, Eotaxin-3, Decorin, α-SMA, MCLK, I-TAC, EGFR und TIMP-1 ausgewählt sind;
(ii) Kontaktieren des Mittels mit dermalen Fibroblastenzellen, die alleine oder in Gegenwart von Lungenepithelzellen mit zumindest zwei Faktoren, die aus IL-1β, TNF-α und IFNγ ausgewählt sind, kultiviert wurden; und das Aufzeichnen von Veränderungen in zumindest drei verschiedenen Parametern als Parameterablesungen als Folge der Einbringung des Mittels, wobei die Parameter aus ICAM, VCAM, CD40, CD90, IP-10, MCP-1, Collagen I, Mig, m-CSF, TIMP-2, PAI-I, IL-8, Collagen III, HLA-DR, MMP-1, MMP-9, Proliferation, TGF-β1, Eotaxin-3, Decorin, α-SMA, MCLK, I-TAC, EGFR und TIMP-1 ausgewählt sind;
(iii) Kontaktieren des Mittels mit bronchialen Epithelzellen, die mit TNF-α und IL-4 kultiviert wurden, und das Aufzeichnen von Veränderungen in zumindest drei verschiedenen Parametern als Parameterablesungen als Folge der Einbringung des Mittels, wobei die Parameter aus CD90, Keratin 8/18, Eotaxin-3, I-TAC, ICAM-1, EGFR, IL-1α, IL-8, MCP-1, MMP-9, MMP-1, MMP-3, PAI-1, TGF-β1, TIMP-2, uPA, tPA, CD87, VCAM-1, IP-10, Elafin/SKALP, Endothelin-1, Gro-a, CD119, IL-6, GM-CSF, IL-16, FGF, PDGF, CD44, E-Cadherin, CD40, IL-15Rα, CD1d, CD80, CD86, TARC, Eotaxin-1, CD95, MCP-4 und MIP-3a ausgewählt sind;
(iv) Kontaktieren des Mittels mit bronchialen Epithelzellen, die mit Fibroblasten mit TNF-α und IL-4 kultiviert wurden; und das Aufzeichnen von Veränderungen in zumindest drei verschiedenen Parametern als Parameterablesungen als Folge der Einbringung des Mittels, wobei die Parameter aus CD90, Keratin 8/18, Eotaxin-3, I-TAC, ICAM-1, EGFR, IL-1α, IL-8, MCP-1, MMP-9, MMP-1, MMP-3, PAI-1, TGF-β1, TIMP-2, uPA, tPA, CD87, VCAM-1, IP-10, Elafin/SKALP, Endothelin-1, Gro-a, CD119, IL-6, GM-CSF, IL-16, FGF, PDGF, CD44, E-Cadherin, CD40, IL-15Rα, CD1d, CD80, CD86, TARC, Eotaxin-1, CD95, MCP-4 und MIP-3a ausgewählt sind; oder
(v) Kontaktieren des Mittels mit Monozyten, die mit Fibroblasten in Gegenwart von zumindest zwei Faktoren, die aus TGF-β1, M-CSF, apoptotischen bronchialen Epithelzellen (1:1-Verhältnis mit Monozyten), IL-4, IL-13, IL-6, IFN-γ und GM-CSF ausgewählt sind, kultiviert wurden; und das Aufzeichnen von Veränderungen in zumindest drei verschiedenen Parametern als Parameterablesungen als Folge der Einbringung des Mittels, wobei die Parameter aus TGF-β1, Mannoserezeptor, CD23, CD36, CD68, HLADR, DC-SIGN, CR1, Annexin-1, SAA, CD1a, Cystatin C, FLIP, ADAM15, CD16, CD64, LIGHT, I-309, CD14, CD40, CD69, CD86, CD80, CD163, CD13, E-Selectin, TNF-α, IL1α, IL-1β, IL-6, IL-8, IL-10, IL-12, IL-18, M-CSF, MIP-1a, MIP-3α, Mac-1 (CD11b/CD18), MCP-1, MCP-4, Fibronectin, MDC, MIG, MMP9, MMP13, Plasminogenaktivatorrezeptor vom Urokinasetyp (uPAR, CD87), Gewebefaktor (CD142), Transferrin und VCAM-1 (CD106) ausgewählt sind.

7. Verfahren nach Anspruch 1, wobei ein Kandidatenmittel, das als geeignet für die Entwicklung bei der Behandlung von Fibrose identifiziert wurde, zumindest 10 erwünschte Veränderungen aufweist, die in Tabelle 2 angeführt sind.

8. Verfahren nach Anspruch 6, wobei die Gesamtpunkte zur Bestimmung der Eignung eines Kandidatenmittels für die Entwicklung bei der Behandlung von Fibrose negativ für Parameteränderungen, wie sie in Tabelle 3 angeführt sind, ausfallen.

## Revendications

1. Procédé d'identification d'agents qui stabilisent ou inversent la fibrose, le procédé consistant à :
mettre en contact l'agent avec des cellules de fibroblastes dermiques humaines cultivées seules ou en présence de cellules épithéliales pulmonaires, avec au moins cinq facteurs sélectionnés parmi le TNF, l'IL-1, l'IFNγ, l'EGF, le bFGF + HSPG, et le PDGFbb agissant sur les cellules ;
enregistrer les modifications d'au moins trois paramètres, en tant que mesures des paramètres, suite à l'introduction de l'agent, où les paramètres sont sélectionnés parmi ICAM, VCAM, CD40, CD90, IP-10, MCP-1, le collagène 1, Mig, le m-CSF, TIMP-2, PAI-I, l'IL-8, le collagène III, HLA-DR, MMP-1, MMP-9, une prolifération, le TGF-β1, l'éotaxine-3, la décorine, alpha-SMA, MLCK, I-TAC, l'EGFR et TIMP-1 ;
dériver un profil d'un ensemble de données biologiques à partir des modifications des mesures des paramètres, où le profil de l'ensemble de données biologiques comporte des données qui sont normalisées pour être un rapport de données de test sur des données de contrôle sur le même type cellulaire dans des conditions de contrôle en l'absence de l'agent ; et
analyser le profil de l'ensemble de données biologiques par un algorithme de reconnaissance de motif à paramètres multiples afin de quantifier le lien entre le profil de l'ensemble de données biologiques et des profils d'ensembles de données biologiques de référence qui comprennent des agents connus qui ciblent des voies spécifiques, où la présence ou l'absence de lien avec ledit profil de l'ensemble de données biologiques de référence offre une caractérisation du mécanisme d'action dudit agent,
ce qui permet ainsi d'identifier l'agent comme un agent potentiel qui stabilise ou inverse la fibrose.

2. Procédé selon la revendication 1, dans lequel l'agent est un agent génétique.

3. Procédé selon la revendication 1, dans lequel l'agent est un agent chimique ou biologique.

4. Procédé selon la revendication 1, dans lequel au moins un deuxième profil d'un ensemble de données biologiques est concaténé.

5. Procédé selon la revendication 4, dans lequel au moins un troisième profil d'un ensemble de données biologiques est concaténé.

6. Procédé selon la revendication 4, dans lequel le deuxième profil d'un ensemble de données biologiques est dérivé :
(i) d'une mise en contact de l'agent avec des cellules de fibroblastes dermiques cultivées seules ou en présence de cellules épithéliales pulmonaires avec au moins deux facteurs sélectionnés parmi le TGFβ, le TNFα, l'IL-4 et l'IGF2, agissant sur les cellules ; et d'un enregistrement des modifications d'au moins trois paramètres différents, en tant que mesures des paramètres, suite à l'introduction de l'agent, où les paramètres sont sélectionnés parmi ICAM, VCAM, CD40, CD90, IP-10, MCP-1, le collagène 1, Mig, le m-CSF, TIMP-2, PAI-I, l'IL-8, le collagène III, HLA-DR, MMP-1, MMP-9, une prolifération, le TGF-β1, l'éotaxine-3, la décorine, alpha-SMA, MLCK, I-TAC, l'EGFR et TIMP-1 ;
(ii) d'une mise en contact de l'agent avec des cellules de fibroblastes dermiques cultivées seules ou en présence de cellules épithéliales pulmonaires avec au moins deux facteurs sélectionnés parmi l'IL-1β, le TNF-α et l'IFN-γ ; et d'un enregistrement des modifications d'au moins trois paramètres différents, en tant que mesures des paramètres, suite à l'introduction de l'agent, où les paramètres sont sélectionnés parmi ICAM, VCAM, CD40, CD90, IP-10, MCP-1, le collagène 1, Mig, le m-CSF, TIMP-2, PAI-I, l'IL-8, le collagène III, HLA-DR, MMP-1, MMP-9, une prolifération, le TGF-β1, l'éotaxine-3, la décorine, alpha-SMA, MLCK, I-TAC, l'EGFR et TIMP-1 ;
(iii) d'une mise en contact de l'agent avec des cellules épithéliales bronchiques cultivées avec le TNF-α et l'IL-4 ; et d'un enregistrement des modifications d'au moins trois paramètres différents, en tant que mesures des paramètres, suite à l'introduction de l'agent, où les paramètres sont sélectionnés parmi CD90, la kératine 8/18, l'éotaxine-3, I-TAC, ICAM-1, l'EGFR, l'IL-1α, l'IL-8, MCP-1, MMP-9, MMP-1, MMP-3, PAI-1, le TGF-β1, TIMP-2, uPA, tPA, CD87, VCAM-1, IP-10, l'élafine/SKALP, l'endothéline-1, Gro-a, CD119, l'IL-6, le GM-CSF, l'IL-16, le FGF, le PDGF, CD44, l'E-cadhérine, CD40, l'IL-15Rα, CD1d, CD80, CD86, TARC, l'éotaxine-1, CD95, MCP-4 et MIP-3a ;
(iv) d'une mise en contact de l'agent avec des cellules épithéliales bronchiques cultivées avec des fibroblastes avec le TNF-α et l'IL-4 ; et d'un enregistrement des modifications d'au moins trois paramètres différents, en tant que mesures des paramètres, suite à l'introduction de l'agent, où les paramètres sont sélectionnés parmi CD90, la kératine 8/18, l'éotaxine-3, I-TAC, ICAM-1, l'EGFR, l'IL-1α, l'IL-8, MCP-1, MMP-9, MMP-1, MMP-3, PAI-1, le TGF-β1, TIMP-2, uPA, tPA, CD87, VCAM-1, IP-10, l'élafine/SKALP, l'endothéline-1, Gro-a, CD119, l'IL-6, le GM-CSF, l'IL-16, le FGF, le PDGF, CD44, l'E-cadhérine, CD40, l'IL-15Rα, CD1d, CD80, CD86, TARC, l'éotaxine-1, CD95, MCP-4 et MIP-3a ; ou
(v) d'une mise en contact de l'agent avec des monocytes cultivés avec des fibroblastes en présence d'au moins deux facteurs sélectionnés parmi le TGF-β1 ; le M-CSF ; des cellules épithéliales bronchiques apoptotiques (rapport 1:1 avec les monocytes) ; l'IL-4, l'IL-13, l'IL-6, l'IFN-γ ; et le GM-CSF, et d'un enregistrement des modifications d'au moins trois paramètres différents, en tant que mesures des paramètres, suite à l'introduction de l'agent, où les paramètres sont sélectionnés parmi le TGF-β1, le récepteur du mannose, CD23, CD36, CD68, HLADR, DC-SIGN, CR1, l'annexine-1, SAA, CD1a, la cystatine C, FLIP, ADAM15, CD16, CD64, LIGHT, 1-309, CD14, CD40, CD69, CD86, CD80, CD163, CD13, l'E-sélectine, le TNF-alpha, l'IL-1alpha, l'IL-1bêta, l'IL-6, l'IL-8, l'IL-10, l'IL-12, l'IL-18, le M-CSF, MIP-1a, MIP-3alpha, Mac-1 (CD11b/CD18), MCP-1, MCP-4, la fibronectine, MDC, MIG, MMP9, MMP13, le récepteur de l'activateur du plasminogène de type urokinase (uPAR, CD87), le facteur tissulaire (CD142), la transferrine et VCAM-1 (CD106).

7. Procédé selon la revendication 1, dans lequel un agent candidat identifié comme étant approprié pour un développement dans le traitement de la fibrose correspond à au moins 10 modifications souhaitées décrites dans le Tableau 2.

8. Procédé selon la revendication 6, dans lequel le score total pour déterminer l'adéquation d'un agent candidat pour un développement dans le traitement de la fibrose attribue un score négatif à une modification de paramètre tel que décrit dans le Tableau 3.
